# EUROPEAN PATENT APPLICATION

(11) **EP 3 744 851 A1**
(43) Date of publication of application: **02.12.2020**
(21) Application number: 19740980.8
(22) Date of filing: 16.01.2019
(51) Int. Cl.: C12P 5/02, B01D 11/04, C12M 1/00, C12N 1/19, C12N 1/21, B81B 1/00

(54) **TARGET COMPONENT EXTRACTION METHOD, EXTRACTION DEVICE, PRODUCTION METHOD AND PRODUCTION DEVICE**

(30) Priority: 22.01.2018 JP 2018008273
(71) Applicant: AJINOMOTO CO., INC., Chuo-ku Tokyo 104-8315 (JP)
(72) Inventor: HIOKI, Satoshi, Kawasaki-shi, Kanagawa 210-8681 (JP); NAKAJIMA, Ayumi, Kawasaki-shi, Kanagawa 210-8681 (JP); NITTA, Nobuhisa, Kawasaki-shi, Kanagawa 210-8681 (JP)
(74) Representative: Hoffmann Eitle
(86) International application number: PCT/JP2019/001140
(87) International publication number: WO 2019/142832

(57) **Abstract**

An extraction method is aimed at extracting an object component by transferring the object component from a first liquid containing a microorganism and the object component to a second liquid to which the object component can be transferred and that is capable of causing phase separation from the first liquid, with use of a flow channel including a merging part, a flow part, and an outlet part. The extraction method includes a step of supplying the first liquid to the merging part, a step of supplying the second liquid to the merging part, a step of passing the first liquid and the second liquid supplied to the merging part, through the flow part, in a state where a phase of the first liquid and a phase of the second liquid are separated from each other, a step of discharging the first liquid and the second liquid passed through the flow part, from the outlet part, and a step of collecting the first liquid and the second liquid discharged from the outlet part, into a collector.

## Description

### Field of the Invention

The present invention relates to an extraction method and an extraction apparatus for an object component from a liquid containing a microorganism and the object component. The present invention relates to a production method and a production apparatus for the object component using a microorganism.

### Description of the Related Art

A production method using a microorganism may be employed to produce an object component. In such a production method, the object component is generally generated by a microorganism in a culture medium containing the microorganism. Then, sterilization is carried out as required, and the microorganism is separated, before the object component is separated from the culture medium. As a method for separating the object component from the culture medium, an extraction method may be employed.

Techniques described in Non Patent Literatures 1 to 8 are known.

### Related Art Reference

### Non Patent Literature

Non Patent Literature 1: Kai Wang, Guangsheng Luo "Microflow extraction: A review of recent development" Chemical Engineering Science (2017) 169, pp. 18-33
Non Patent Literature 2: Mercedes C. Morales, Jeffrey D. Zahn "Droplet enhanced microfluidic-based DNA purification from bacterial lysates via phenol extraction" Microfluid Nanofluid (2010) 9, pp. 1041-1049
Non Patent Literature 3: Ki-Hwan Nam, Woo-Jin Chang, Hyejin Hong, Sang-Min Lim, Dong-Il Kim, Yoon-Mo Kool "Continuous-Flow Fractionation of Animal Cells in Microfluidic Device Using Aqueous Two-Phase Extraction" Biomedical Microdevices (2005) 7: 3, pp. 189-195
Non Patent Literature 4: Masatoshi Tsukamoto, Shu Taira, Shohei Yamamura, Yasutaka Morita, Naoki Nagatani, Yuzuru Takamura, Eiichi Tamiya "Cell separation by an aqueous two-phase system in a microfluidic device" The Royal Society of Chemistry (2009) 134, pp. 1994-1998
Non Patent Literature 5: Najla Ben Akacha, Mohamed Gargouri "Microbial and enzymatic technologies used for the production of natural aroma compounds: Synthesis, recovery modeling, and bioprocesses" food and bioproducts processing (2015) 94, pp. 675-706
Non Patent Literature 6: Hendrik Schewe, Marco Antonio Mirata and Jens Schrader "Bioprocess Engineering for Microbial Synthesis and Conversion of Isoprenoids" Adv Biochem Eng Biotechnol (2015) 148, pp. 251-286
Non Patent Literature 7: Arjan S. Heeres, Carolina S. F. Picone, Luuk A. M. van der Wielen, Rosiane L. Cunha, and Maria C. Cuellar "Microbial advanced biofuels production: overcoming emulsification challenges for large-scale operation" Trends in Biotechnology, April 2014, Vol 32, No. 4, pp. 221-229
Non Patent Literature 8: Jovan Jovanovic, Evgeny V. Rebrov, T. A. (Xander) Nijhuis, M. T. Kreutzer, Volker Hessel, and Jaap C. Schouten, "Liquid-Liquid Flow in a Capillary Microreactor: Hydrodynamic Flow Patterns and Extraction Performance" Industrial & Engineering Chemistry Research, 2012, 51, pp. 1015-1026

### SUMMARY OF THE INVENTION

### Problem to be solved by the Invention

The separation of a microorganism from a culture medium requires great effort and time. Therefore, when the separation of a microorganism and the extraction of an object component are performed in separate steps, the cost tends to increase. Therefore, the inventors of the present invention have studied in order to simultaneously perform the separation of the microorganism and the extraction of the object component.

The inventors of the present invention have studied to simultaneously perform the separation of a microorganism and the extraction of an object component by putting a culture medium and an extraction solvent into a container and then stirring. However, in most of experiments, the stirring causes suspension, and thus the microorganism cannot be separated. In some experiments, a culture medium containing a microorganism can separate from an extraction liquid not containing the microorganism by leaving the container to stand after the stirring. However, this separation requires several minutes or more for leaving it to stand, and cannot accomplish a short-time implementation.

The present invention has been created in view of the problem mentioned above. The present invention has an object to provide: an extraction method and an extraction apparatus capable of simultaneously performing separation of a microorganism and extraction of an object component; and a production method and a production apparatus capable of producing the object component while simultaneously performing the separation of microorganism and the extraction of the object component.

### Means for Solving the Problem

[1] An extraction method for extracting an object component by transferring the object component from a first liquid containing a microorganism and the object component to a second liquid to which the object component is transferrable and that is capable of causing phase separation from the first liquid, with use of a flow channel including a merging part, a flow part, and an outlet part, the extraction method comprising:
   a step of supplying the first liquid to the merging part;
   a step of supplying the second liquid to the merging part;
   a step of passing the first liquid and the second liquid supplied to the merging part through the flow part, in a state where a phase of the first liquid and a phase of the second liquid are separated from each other;
   a step of discharging the first liquid and the second liquid having passed through the flow part, from the outlet part; and
   a step of collecting the first liquid and the second liquid having been discharged from the outlet part, into a collector.
[2] The extraction method according to [1], wherein, in the flow part, the first liquid and the second liquid form slug flow with an alternate alignment of the phase of the first liquid and the phase of the second liquid in a flowing direction.
[3] The extraction method according to [1] or [2], wherein the microorganism has an ability to generate the object component.
[4] The extraction method according to any one of [1] to [3], wherein the microorganism is a genetically modified microorganism.
[5] The extraction method according to any one of [1] to [4], wherein the first liquid is a culture medium containing the microorganism and the object component, and the second liquid contains an organic solvent.
[6] The extraction method according to any one of [1] to [5], wherein the object component is a hydrophobic substance.
[7] The extraction method according to any one of [1] to [6], wherein the object component is an isoprenoid compound or a vanilloid compound.
[8] The extraction method according to any one of [1] to [7], wherein the microorganism is a bacterium or a yeast.
[9] The extraction method according to [8], wherein the bacterium is one selected from the group consisting of Corynebacterium bacterium, Pantoea bacterium, Bacillus bacterium, Escherichia bacterium, and Enterobacter bacterium.
[10] The extraction method according to any one of [1] to [9], wherein a concentration of the object component in the first liquid supplied to the merging part is 0.1 g/L or more and 500 g/L or less.
[11] The extraction method according to any one of [1] to [10], wherein a dry microorganism weight of the first liquid supplied to the merging part is 0.1 g/L or more and 500 g/L or less.
[12] The extraction method according to any one of [1] to [11], wherein the flow part has an inner diameter of 0.25 mm or more and 10.0 mm or less.
[13] The extraction method according to any one of [1] to [12], wherein temperatures of the first liquid and the second liquid in the flow part are 10°C or more and 90°C or less.
[14] An extraction apparatus for extracting an object component by transferring the object component from a first liquid containing a microorganism and the object component to a second liquid to which the object component is transferrable and that is capable of causing phase separation from the first liquid, the extraction apparatus comprising:
   a first supplying portion that can supply the first liquid;
   a second supplying portion that can supply the second liquid;
   a flow channel including
      a merging part to which the first liquid and the second liquid can be supplied,
      a flow part through which the first liquid and the second liquid supplied to the merging part can pass, and
      an outlet part from which the first liquid and the second liquid passed through the flow part can be discharged; and
   a collector that can collect the first liquid and the second liquid discharged from the outlet part, wherein
   the first liquid and the second liquid can pass through the flow part in a state where a phase of the first liquid and a phase of second liquid are separated from each other.
[15] A production method for producing an object component, the production method comprising:
   a step of generating the object component by a microorganism in a first container that stores therein a first liquid containing the microorganism;
   a step of supplying the first liquid from the first container to a merging part of a flow channel that includes the merging part, a flow part, and an outlet part;
   a step of supplying a second liquid to which the object component is transferrable and that is capable of causing phase separation from the first liquid, to the merging part;
   a step of passing the first liquid and the second liquid supplied to the merging part through the flow part, in a state where a phase of the first liquid and a phase of the second liquid are separated from each other;
   a step of discharging the first liquid and the second liquid having passed through the flow part, from the outlet part; and
   a step of collecting the first liquid and the second liquid having been discharged from the outlet part, into a collector.
[16] The production method for the object component according to [15], including a step of returning the first liquid collected in the collector to the first container.
[17] The production method for the object component according to [15] or [16], including a step of returning the second liquid collected in the collector to the merging part.
[18] The production method for the object component according to any one of [15] to [17], wherein the object component has a property of inhibiting the microorganism from generating the object component.
[19] A production apparatus for producing an object component, the production apparatus comprising:
   a first container that can store a first liquid containing a microorganism and can allow the microorganism to generate the object component;
   a first supplying portion that can supply the first liquid stored in the first container;
   a second supplying portion that can supply a second liquid to which the object component is transferrable and that is capable of causing phase separation from the first liquid;
   a flow channel including
      a merging part to which the first liquid and the second liquid can be supplied,
      a flow part through which the first liquid and the second liquid supplied to the merging part can pass, and
      an outlet part from which the first liquid and the second liquid passed through the flow part can be discharged; and
   a collector that can collect the first liquid and the second liquid discharged from the outlet part, wherein
   the first liquid and the second liquid can pass through the flow part in a state where a phase of the first liquid and a phase of second liquid are separated from each other.
[20] The production apparatus for the object component according to [19], comprising a first circulation portion that can return the first liquid collected in the collector to the first container.

### Advantageous Effects of Invention

According to the present invention, it is possible to provide: an extraction method and an extraction apparatus capable of simultaneously performing separation of a microorganism and extraction of an object component; and a production method and a production apparatus capable of producing the object component while simultaneously performing the separation of microorganism and the extraction of the object component.

### Brief Description of Drawings

FIG. 1 is a schematic diagram schematically illustrating an extraction apparatus according to a first embodiment of the present invention.
FIG. 2 is a cross-sectional view schematically illustrating a cross section of an extraction duct used in the first embodiment of the present invention.
FIG. 3 is a cross-sectional view schematically illustrating a flow part for illustrating an example of a slug flow formed by a first liquid and a second liquid.
FIG. 4 is a schematic diagram schematically illustrating a production apparatus according to a second embodiment of the present invention.
FIG. 5 is a photograph of a collector taken right after the first liquid and the second liquid are collected in Example 59 of the present invention.
FIG. 6 is a photograph of a collector taken right after the first liquid and the second liquid are collected in Example 65 of the present invention.
FIG. 7 is a photograph of a collector taken right after the first liquid and the second liquid are collected in Example 73 of the present invention.
FIG. 8 is a photograph of a tube container taken right after stirring is completed in Comparative Example 4.
FIG. 9 is a photograph of the tube container taken at 20 minutes after stirring is completed in Comparative Example 4.
FIG. 10 is a photograph of the tube container taken at 1 hour after stirring is completed in Comparative Example 4.
FIG. 11 is a photograph of the tube container taken at 24 hours after stirring is completed in Comparative Example 4.
FIG. 12 is a photograph of the tube container taken at 60 hours after stirring is completed in Comparative Example 5.
FIG. 13 is a graph illustrating a concentration of linalool in isopropyl myristate measured in Example 74 of the present invention.
FIG. 14 is a graph illustrating total generated amounts of linalool contained in a culture medium and linalool contained in isopropyl myristate in Example 74 of the present invention and Comparative Example 13.
FIG. 15 is a graph illustrating concentration of an organic acid in the culture medium at 24.5 hours after the beginning of culturing in Example 74 of the present invention and Comparative Example 13.
FIG. 16 is a graph illustrating the concentration of linalool in isopropyl myristate measured in Example 75 of the present invention.
FIG. 17 is a graph illustrating the concentration of linalool in the culture medium in Example 75 of the present invention and Comparative Examples 14-16.
FIG. 18 is a graph illustrating total generated amounts of linalool contained in the culture medium and linalool contained in isopropyl myristate in Example 75 of the present invention and Comparative Examples 14-16.
FIG. 19 is a graph illustrating concentration of the organic acid in the culture medium at 43 hours after the beginning of culturing in Example 75 of the present invention and Comparative Examples 14-16.

### Description of Embodiments

Hereinafter, the present invention is described in detail below with reference to embodiments and examples. The present invention is not limited to the embodiments and the examples described below, and can be carried out with any modifications made to the extend not departing from the claims of the present invention and the equivalents thereof.

### [1. First embodiment]

In the extraction method of the present invention, an object component is extracted by transferring from a first liquid containing a microorganism and the object component to a second liquid to which the object component can be transferred. As the second liquid, a liquid capable of causing phase separation from the first liquid in an appropriate condition is used. Usually, the first liquid contains a first solvent, and the second liquid contains a second solvent. The combination of the first solvent and the second solvent achieves the phase separation in an appropriate condition, thereby enabling the phase separation between the first liquid and the second liquid. In general, water is often used as the first solvent while an organic solvent capable of causing the phase separation from water is often used as the second solvent. The combination of the first solvent and the second solvent is not limited to this combination.

Hereinafter, an embodiment of the extraction method is described with reference to drawings.

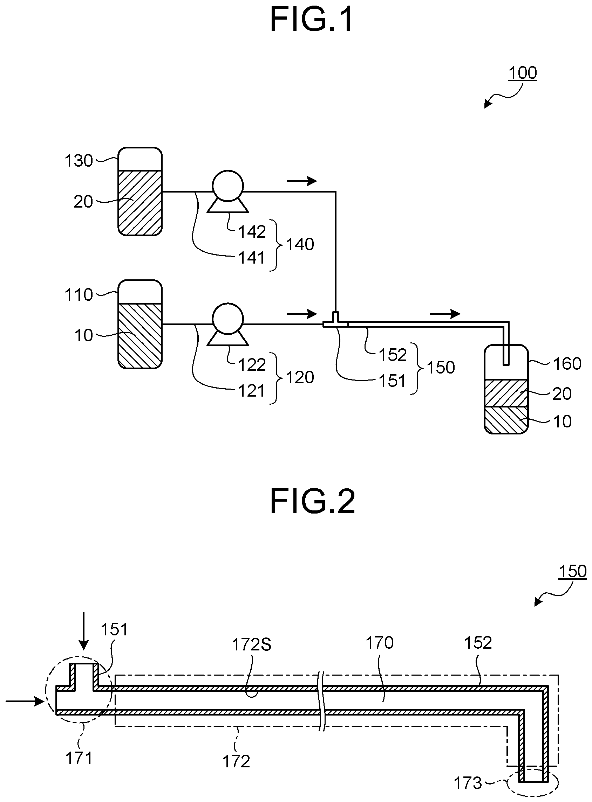

FIG. 1 is a schematic diagram schematically illustrating an extraction apparatus 100 according to a first embodiment of the present invention.

As illustrated in FIG. 1, the extraction apparatus 100 according to the first embodiment of the present invention is an apparatus for extracting the object component (not illustrated) by transferring it from the first liquid 10 to the second liquid 20, and comprises a first container 110, a first supplying portion 120, a second container 130, a second supplying portion 140, an extraction duct 150 having a flow channel (see FIG. 2) therein, and a collector 160.

The first container 110 is a container storing therein the first liquid 10 to be supplied to the flow channel inside the extraction duct 150. The first liquid 10 stored in the first container 110 refers to an original liquid that is present before the extraction of object component. The first liquid 10 contains the microorganism, the object component and the first solvent.

The first container 110 may have a volume capacity of at least 100 liters. In some embodiments, the container has a volume capacity of at least 1000 liters, at least 10,000 liters, at least 50,000 liters, at least 100,000 liters, or at least 250,000 liters.

A type of microorganism is not limited. The microorganism may be used alone or in any combination of two or more types thereof.

The concentration of microorganism in the first liquid 10 that is stored in the first container 110 is not particularly limited. Preferred ranges are as follows. A dry weight of microorganism of the first liquid 10 is usually 0.01 g/L or more, preferably 0.1 g/L or more, more preferably 1 g/L or more, and particularly preferably 5 g/L or more, and is usually 500 g/L or less, preferably 300 g/L or less, and more preferably 200 g/L or less. When the concentration of microorganism is equal to or higher than the aforementioned lower limit, it is possible to utilize advantageous effects capable of separating microorganism. When the concentration of microorganism is equal to or lower than the aforementioned upper limit, it is possible to perform the microorganism separation smoothly.

Generally, the concentration of microorganism in the first liquid 10 can also be represented in terms of O.D. value. For example, an O.D. 600 nm of the first liquid 10 stored in the first container 110 is preferably 0.028 or more, more preferably 2.8 or more, and particularly preferably 28.3 or more, and is preferably 1415 or less, more preferably 840 or less, and particularly preferably 560 or less. For example, an O.D. 562 nm of the first liquid 10 stored in the first container 110 is preferably 0.03 or more, more preferably 3 or more, and particularly preferably 30 or more, and is preferably 1500 or less, more preferably 900 or less, and particularly preferably 600 or less. When the O.D. value of the first liquid 10 is equal to or higher than the aforementioned lower limit, it is possible to utilize advantageous effects capable of separating microorganism. When the concentration of microorganism is equal to or lower than the aforementioned upper limit, it is possible to perform the microorganism separation smoothly.

The O.D. 600 nm can be measured at a measurement wavelength of 600 nm by using a spectrophotometer (for example, "Hitachi U-2900" manufactured by Hitachi High-Tech Science Corporation) as a measurement device. The O.D. 562 nm can be measured at a measurement wavelength of 562 nm using the aforementioned measurement device.

A type of object component is not limited. The object component may be used alone or in any combination of two or more types thereof.

The concentration of the object component in the first liquid 10 stored in the first container 110 is not particularly limited. Preferred ranges are as follows. The concentration of the object component is usually 0.1 g/L or more, preferably 0.3 g/L or more, and more preferably 0.5 g/L or more, and is usually 500 g/L or less, preferably 50 g/L or less, more preferably 30 g/L or less, and particularly preferably 20 g/L or less. When the concentration of the object component is equal to or higher than the aforementioned lower limit, it is possible to perform the separation of object component smoothly. When the concentration of object component is equal to or lower than the aforementioned upper limit, it is possible to perform the separation of object component smoothly.

The concentration of the object component in the first liquid 10 can be determined by gas chromatography (hereinafter appropriately referred to as "GC").

The first solution 10 usually includes the first solvent. The first solvent is preferably capable of causing phase separation from the second solvent contained in the second solution 20. The first solvent preferably has a low solubility of the object component. The type of the first solvent is preferably selected as appropriate according to the types of the object component and the second solvent. Examples of preferred first solvent include aqueous solvents such as water. The first solvent may be used alone or in any combination of two or more types thereof.

The pH of the first liquid 10 may be controlled in an appropriate range. When the pH of the first liquid 10 is controlled appropriately, it is possible to sterilize and culture the microorganism. In some liquids used for the culture of microorganism, components such as the microorganism, surfactant substance produced by the microorganism and defoaming agent used during cultivation may exhibit a surfactant-like activity to prevent the first liquid 10 and the second liquid 20 from causing the phase separation. However, with the appropriate control of pH, it is possible to suppress such a surfactant-like activity. The specific pH of the first liquid 10 is usually 1.0 or more, preferably 2.0 or more, and more preferably 2.5 or more, and is usually 10.0 or less, preferably 9.0 or less, and more preferably 8.0 or less.

The first liquid 10 may contain an optional component other than the microorganism, the object component and the first solvent, if necessary, as long as it does not significantly interfere with the separation of the microorganism and the extraction of the object component.

The first supplying portion 120 is a device provided in such a way as to be able to supply the first liquid 10 stored in the first container 110 to a flow channel (see FIG. 2) in the extraction duct 150. In an example illustrated for this embodiment, the first supplying portion 120 comprises a first supplying duct 121 as a first supplying channel connected to the first container 110 and the extraction duct 150, and a first liquid feeding pump 122 as a first flow amount controlling portion provided in the first supplying duct 121. The first supplying portion 120 is provided such that the first liquid 10 stored in the first container 110 can be supplied to the flow channel in the extraction duct 150 through the first supplying duct 121. The first supplying portion 120 is provided such that the flow amount of the first liquid 10 supplied through the first supplying duct 121 can be controlled by the first liquid feeding pump 122.

The second container 130 is a container for storing therein the second liquid 20 to be supplied to a flow channel in the extraction duct 150. As the second liquid 20 stored in the second container 130, a liquid to which the object component is transferable and that is capable of causing the phase separation from the first liquid 10 is used in order to extract the object component included in the first liquid 10.

The capability of transferring the object component to the second liquid 20 refers to the capability of transferring the object component contained in the first liquid 10 to the second liquid 20 when the first liquid 10 and the second liquid 20 pass through the flow channel in the extraction duct 150. The transfer is usually carried out by dissolving the object component into the second solvent contained in the second liquid 20.

The capability of phase-separating the second liquid 20 from the first liquid 10 refers to the capability of separating the phase of the first liquid 10 and the phase of the second liquid 20 from each other when the first liquid 10 and the second liquid 20 pass through the flow channel in the extraction duct 150. As long as the phase of the first liquid 10 and the phase of the second liquid can be separated from each other, a part of the second solvent may be mixed into the phase of the first liquid 10, and a part of the first solvent may be mixed into the phase of the second liquid 20. However, in order to efficiently perform the separation of the microorganism and the extraction of the object component, such a mixing is preferably minimized.

The second liquid 20 usually includes the second solvent. The second solvent preferably has a high solubility of the object component. The second solvent is preferably capable of being phase-separated from the first solvent contained in the first liquid 10. The type of the second solvent is preferably selected as appropriate according to types of the object component and the first solvent contained in the first liquid 10. In the case that the first solvent is water, examples of a preferred second solvent include an organic solvent capable of causing phase separation from water.

Examples of the organic solvent include ether solvents such as methyl-tertiary-butyl ether; ester solvents such as isopropyl myristate and ethyl acetate; ketone solvents; hydrocarbon solvents such as hexane, heptane, and cyclohexane; and vegetable oils.

When the first solvent is water, examples of the preferred second solvent are as follows.

For example, when the object component contains an isoprenoid compound such as linalool, examples of the second solvent include isopropyl myristate, ethyl acetate, methyl-tertiary-butyl ether, hexane, heptane, cyclohexane, and vegetable oil.

For example, when the object component contains an aromatic compound such as vanillin, examples of the second solvent include toluene, ethyl acetate, hexane, heptane, and vegetable oil.

The second solvent may be used alone or in any combination of two or more types thereof.

The second liquid 20 may contain an optional component other than the second solvent, if necessary, as long as it does not significantly interfere with the separation of microorganism and the extraction of the object component.

The second supplying portion 140 is a device provided in such a way as to be able to supply the second liquid 20 stored in the second container 130 to the flow channel (see FIG. 2) in the extraction duct 150. In an example illustrated for this embodiment, the second supplying portion 140 comprises a second supplying duct 141 as a second supplying channel connected to the second container 130 and the extraction duct 150, and a second liquid feeding pump 142 as a second flow amount controlling portion provided in the second supplying duct 141. The second supplying portion 140 is provided such that the second liquid 20 stored in the second container 130 can be supplied to the flow channel in the extraction duct 150 through the second supplying duct 141. The second supplying portion 140 is provided such that the flow amount of the second liquid 20 supplied through the second supplying duct 141 can be controlled by the second liquid feeding pump 142.

The extraction duct 150 is a member having the flow channel inside the extraction duct 150. In this embodiment described with reference to examples, the extraction duct 150 includes a T-shaped mixer part 151 formed at an upstream end part of the extraction duct 150 and a duct part 152 connected to a downstream of the T-shaped mixer part 151.

FIG. 2 is a cross-sectional view schematically illustrating a cross section of the extraction duct 150 used in the first embodiment of the present invention.

As illustrated in FIG. 2, a flow channel 170 for passing the first liquid 10 and the second liquid 20 is formed in the extraction duct 150. The flow channel 170 comprises a merging part 171, a flow part 172 and an outlet part 173 in this order from the upstream side. In an example illustrated for this embodiment, the merging part 171 is formed in the mixer part 151 of the extraction duct 150, and the flow part 172 and the outlet part 173 are formed in the duct part 152.

The merging part 171 is provided such that the first liquid 10 can be supplied from the first supplying portion 120. The merging part 171 is provided such that the second liquid 20 can be supplied from the second supplying portion 140. Therefore, the merging part 171 is provided such that the first liquid 10 and the second liquid 20 supplied to the merging part 171 are allowed to merge with each other, thereby enabling to pass the first liquid 10 and the second liquid 20 through the common flow part 172. Usually, the merging part 171 is provided at an upstream end part of the flow part 172 so that the first liquid 10 and the second liquid 20 supplied to the merging part 171 can immediately pass through the flow part 172.

The flow part 172 is provided in contact with the merging part 171 so that the first liquid 10 and the second liquid 20 supplied to the merging part 171 can pass through the flow part 172. Since the first liquid 10 and the second liquid 20 pass through the common flow part 172, the object component can be extracted by transferring from the first liquid 10 to the second liquid 20 during a period of the passing through the flow part 172. The flow part 172 is provided such that the first liquid 10 and the second liquid 20 can pass through the flow part 172 while the phase of the first liquid 10 and the phase of the second liquid 20 are separated from each other.

The size of the flow part 172 is adjusted to enable the flow in which the phase of the first liquid 10 and the phase of the second liquid 20 are separated from each other. From the viewpoint of prompting the transfer of the object component from the first liquid 10 to the second liquid 20 by enlarging an area of an interface between the phase of the first liquid 10 and the phase of the second liquid 20 per the flow amount, the sectional area of the flow part 172 is preferably small. The sectional area of the flow part 172 refers to a sectional area in a plane perpendicular to the flowing direction of the flow channel 170.

The specific sectional area of the flow part 172 is preferably 100 mm² or less, more preferably 50 mm² or less, and still more preferably 10 mm² or less. When the sectional area of the flow part 172 is equal to or less than the aforementioned upper limit, it is possible to form slug flow of the first liquid 10 and the second liquid 20 under an appropriate condition. It is possible to increase the area of phase interface per flow amount and improve an extract ratio of the object component. The sectional area of the flow part 172 is preferably 0.1 mm² or more, more preferably 0.5 mm² or more, and still more preferably 1.0 mm² or more. When the sectional area of the flow part 172 is equal to or more than the aforementioned lower limit, it is possible that separation of the first liquid 10 and the second liquid 20 can quickly proceed in the collector 160. Since the amount of liquid passing through the flow part 172 can be increased, it is possible to increase the extraction amount of the object component.

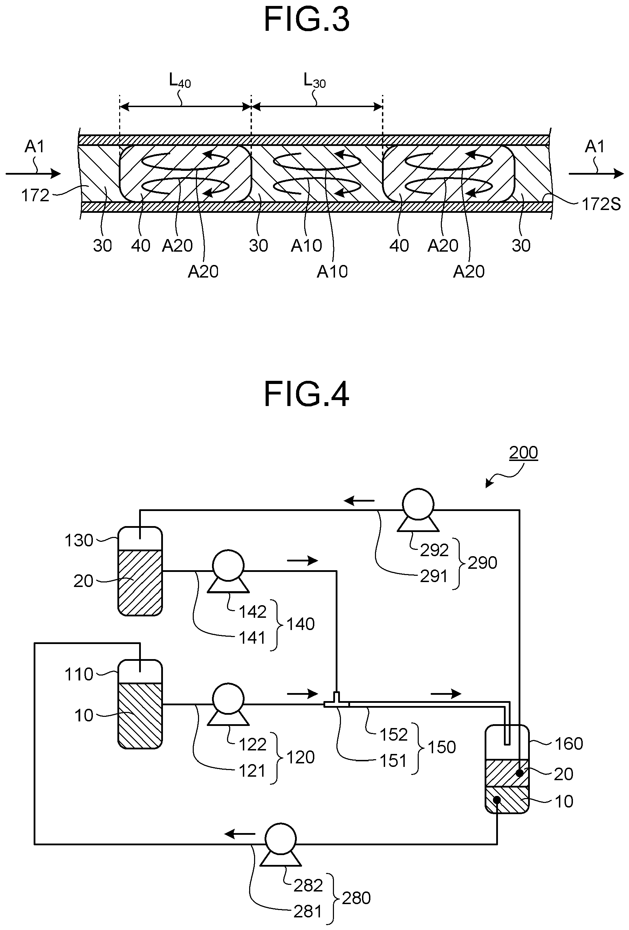

The sectional areas of the flow part 172 may be even or uneven in the flowing direction. For example, when the first liquid 10 and the second liquid 20 form the slug flow in the flow part 172, it may be possible to merge phases (See phase 30 in FIG. 3.) of the first liquid with each other and merge phases (See phase 40 in FIG. 3.) of the second liquid with each other at portions of the flow part 172 with uneven sectional areas in which the sectional area is changed.

The inner diameter of the flow part 172 is preferably 0.25 mm or more, preferably 1.5 mm or more, and may be 2.0 mm or more. The inner diameter of the flow part 172 is preferably 10.0 mm or less, more preferably 8.0 mm or less, and may be 7.0 mm or less. When the inner diameter of the flow part 172 is within the aforementioned range, the separation of microorganism and the extraction of the object component are easily performed smoothly.

A wall surface 172S of the flow part 172 is formed of arbitrarily selected material. For example, an inorganic material such as glass; an alloy such as stainless steel may be used. For example, an organic material such as resin may be used. As a specific material, it is preferred that an appropriate type of material is selected in such a way as to be able to cause the phase separation between the first liquid 10 and the second liquid 20 passing through the flow part 172.

It is preferable that the wall surface 172S of the flow part 172 is a smooth surface without protrusions and recesses. With such a surface, it is possible to suppress generation of turbulent flows resulting from the protrusions or the recesses. Therefore the flow of the first liquid 10 and the second liquid 20 can be stabilized. Thus it is possible to stably maintain the state where the phase of the first liquid 10 and the phase of the second liquid 20 is separated.

The outlet part 173 is an outlet provided so as to deliver the first liquid 10 and the second liquid 20 that have passed through the flow part 172, and is provided at a downstream end of the flow part 172.

As illustrated in FIG. 1, the collector 160 is a container provided in such a way as to be able to collect the first liquid 10 and the second liquid 20 that are discharged through the outlet part 173 of the extraction duct 150. In an example illustrated for this embodiment, the outlet part 173 of the extraction duct 150 is provided to be present inside the collector 160.

With use of the extraction apparatus 100 described above, it is possible to perform an extraction method for extracting the object component by transferring it from the first liquid 10 to the second liquid 20 with use of the flow channel 170 in the extraction duct 150. This extraction method includes:
(1) a step of supplying the first liquid 10 to the merging part 171 of the flow channel 170;
(2) a step of supplying the second liquid 20 to the merging part 171 of the flow channel 170;
(3) a step of passing the first liquid 10 and the second liquid 20 supplied to the merging part 171 through the flow part 172 in a state where the phase of the first liquid 10 and the phase of the second liquid 20 are separated from each other;
(4) a step of discharging the first liquid 10 and the second liquid 20 that has passed through the flow part 172, from the outlet part 173; and
(5) a step of collecting the first liquid 10 and the second liquid 20 discharged from the outlet part 173 into the collector 160.

At the step of supplying the first liquid 10 to the merging part 171, the first liquid 10 stored in the first container 110 is supplied to the merging part 171 of the flow channel 170 in the extraction duct 150 through the first supplying portion 120. Specifically, the first liquid feeding pump 122 is actuated to pump out the first liquid 10 from the first container 110. The pumped first liquid 10 is supplied to the merging part 171 through the first supplying duct 121. The output of the first liquid feeding pump 122 is usually adjusted so as to obtain a linear velocity, a flow amount and a flowing time of the first liquid 10 in the flow part 172 within desired ranges.

At the step of supplying the second liquid 20 to the merging part 171, the second liquid 20 stored in the second container 130 is supplied to the merging part 171 of the flow channel 170 in the extraction duct 150 through the second supplying portion 140. Specifically, the second liquid feeding pump 142 is actuated to pump out the second liquid 20 from the second container 130. The pumped second liquid 20 is supplied to the merging part 171 through the second supplying duct 141. The output of the second liquid feeding pump 142 is usually adjusted so as to obtain a linear velocity, a flow amount and a flowing time of the second liquid 20 in the flow part 172 within desired ranges.

Once the first liquid 10 and the second liquid 20 are supplied to the merging part 171, the first liquid 10 and the second liquid 20 merge. After the merging, the step of passing the first liquid 10 and the second liquid 20 through the flow part 172 is performed. In this step, the first liquid 10 and the second liquid 20 are passed through the flow part 172 without causing suspension of the first liquid 10 and the second liquid 20 while the phase of the first liquid 10 and the phase of the second liquid 20 are separated from each other. At the passing step, the object component is transferred from the first liquid 10 to the second liquid 20 through the interface between the phase of the first liquid 10 and the phase of the second liquid 20. The microorganism contained in the first liquid 10 cannot pass through the interface between the phase of the first liquid 10 and the phase of the second liquid 20, and is kept inside the phase of the first liquid 10. Thus, the phase of the second liquid 20 can selectively extract the object component while suppressing incorporation of the microorganism. In this way, the separation of microorganism and the extraction of object component can simultaneously proceed, the second liquid 20 can be obtained as an extraction liquid containing the second solvent and the object component by passing through the flow part 172.

In order to smoothly separate the phase of the first liquid 10 and the phase of the second liquid 20 after the collection into the collector 160, it is usually required that the phase of the first liquid 10 and the phase of the second liquid 20 are separated from each other at the time of discharging from the outlet part 173. Therefore, it is desirable that the phase of the first liquid 10 and the phase of the second liquid 20 are separated in an entire part of the flow part 172 in the flowing direction. The entire part of the flow part 172 in the flowing direction refers to an area ranging from the merging part 171 to the outlet part 173 in the flow channel 170.

Laminar flow, annular flow, or a slug flow may be one of examples of the flow that is formed by the first liquid 10 and the second liquid 20 while the phase of the first liquid 10 and the phase of the second liquid 20 are separated from each other. In some embodiment, the types of the flow may be determined by a length L of the flow part 172 and the relation of a Weber number of the first liquid 10 and a Weber number of the second liquid 20. The Weber number We is a dimensionless number that is expressed by an equation of "We = d × D × V²/σ", in which "d" represents a density of liquid, "D" represents an inner diameter of the flow part 172, "V" represents a linear velocity of the liquid, and "σ" represents a surface tension of the liquid. Since it is possible to transfer the object component from the phase of the first liquid 10 to the phase of the second liquid 20 when the phase of the first liquid 10 and the phase of the second liquid 20 are separated from each other, any of the specific types of flow may be possible. Among them, the slug flow is preferred from the viewpoint of prompting the transfer of the object component from the first liquid 10 to the second liquid 20.

FIG. 3 is a cross-sectional view schematically illustrating the flow part 172 for illustrating an example of the slug flow formed by the first liquid 10 and the second liquid 20.

As illustrated in FIG. 3, the slug flow is a flow in which the phases 30 of the first liquid and the phases 40 of the second liquid are alternately aligned in the flowing direction represented by an arrow A1. In the slug flow, each of the phases 30 and the phases 40 are formed to a large extent to fill the space inside the flow part 172 by acting like a plug, enabling the alternate alignment of the phases 30 of the first liquid and the phases 40 of the second liquid.

When the slug flow is formed, circulating flows are usually generated as represented by arrows A10 in the phases 30 of the first liquid. Circulating flows are usually generated as represented by arrows A20 in the phases 40 of the second liquid. In the slug flow, the action of the circulating flows enables efficient transfer of the object component from the phase 30 of the first liquid to the phase 40 of the second liquid.

While passing through the flow part 172, a plural of the phases 30 of the first liquid 10 may be combined with each other. While passing through the flow part 172, a plural of the phases 40 of the second liquid 20 may be combined with each other. For example, when an appropriate enlarged diameter part (not illustrated) having a larger sectional area than an upstream part of the enlarged diameter part is formed in the flow part 172, it is possible to cause the combining at the enlarged diameter part.

The passing condition of the first liquid 10 and the second liquid 20 that pass through the flow part 172 can be controlled to such an extent as to cause a state such as the slug flow where the phase 30 of the first liquid and the phase 40 of the second liquid are separated from each other. It is desired that specific conditions are controlled as appropriate according to factors such as a composition of the first liquid 10, a composition of the second liquid 20, a material of the wall surface 172S of the flow part 172, and the size of the flow part 172.

In general, from the viewpoint of promoting transfer of the object component from the first liquid 10 to the second liquid 20 in order to achieve a high extraction rate, the linear velocities of the first liquid 10 and the second liquid 20 are preferably high. The linear velocity refers to a transfer distance per unit time in the flowing direction. From the viewpoint of achieving the high extraction rate, the flowing time of the first liquid 10 and the second liquid 20 is preferably long. The flowing time refers to a time required for flowing from the starting point to ending point of the flow part 172. When the combination of the first liquid 10 and the second liquid 20 easily eliminates the phase-separation, it is desired that upper limits of the linear velocity and the flowing time are controlled as appropriate. With the appropriate control of the upper limits, it is possible to efficiently suppress generation of an intermediate layer resulting from emulsification or the like of the first liquid 10 and the second liquid 20. Therefore, it is possible to increase the amount of the second liquid 20 obtained as the extraction liquid in the collector 160 and improve the separability.

The specific linear velocities of the first liquid 10 and the second liquid 20 in the flow part 172 are usually 1 mm/second or more, preferably 5 mm/second or more, and more preferably 10 mm/second or more. When the linear velocity is equal to or higher than the lower limit, the extraction ratio of the object component can be effectively increased. The upper limit of the linear velocity is different depending on factors such as the composition of the first liquid 10, the composition of the second liquid 20, the material of the wall surface 172S of the flow part 172, and the size of the flow part 172. For example, the upper limit may be 2000 mm/second or less, 1500 mm/seconds or less, 1000 mm/second or less, 600 mm/second or less, 400 mm/seconds or less, 200 mm/second or less, 100 mm/second or less, or 50 mm/second or less.

The specific flowing time of the first liquid 10 and the second liquid 20 in the flow part 172 is usually 0.1 seconds or more, preferably 0.5 seconds or more, more preferably 1 second or more, and still more preferably 2 seconds or more. When the flowing time is equal to or higher than the lower limit, the extraction ratio of the object component can be effectively increased. The upper limit of the flowing time is different depending on factors such as the composition of the first liquid 10, the composition of the second liquid 20, the material of the wall surface 172S of the flow part 172, and the size of the flow part 172. For example, the upper limit may be 1000 seconds or less, 500 seconds or less, 300 seconds or less, 200 seconds or less, 150 seconds or less, 100 seconds or less, or 50 seconds or less.

In one embodiment, from the viewpoint of forming a stable slug flow, a parameter "(L/S)×V" is preferably 1.3 × 10⁶ (s⁻¹) or less, and more preferably 1.2 × 10⁶ (s⁻¹) or less. S represents the sectional area of the flow part 172, L represents the length of the flow part 172, and V represents the linear velocity of the first liquid 10 and the second liquid 20 in the flow part 172.

The amounts of the first liquid 10 and the second liquid 20 that pass through the flow part 172 may be the same or different. Thus, the amount per unit time of the first liquid 10 supplied to the flow part 172 and the amount per unit time of the second liquid 20 supplied to the flow part 172 may be the same or different.

when the slug flow is formed, a length L₃₀ of each phase 30 of the first liquid in the flow part 172 is usually 1 mm or more, preferably 2 mm or more, and more preferably 3 mm or more. When the length L₃₀ of the phase 30 of the first liquid is equal to or higher than the aforementioned lower limit, the separation between the first liquid 10 and the second liquid 20 in the collector 160 can proceed quickly. The upper limit of the length L₃₀ of the phase 30 of the first liquid is usually 5 cm or less, preferably 2 cm or less, and more preferably 1 cm or less. When the length L₃₀ of the phase 30 of the first liquid is equal to or lower than the aforementioned upper limit, the transfer of the object component from the first liquid 10 to the second liquid 20 can be facilitated.

When the slug flow is formed, the length L₄₀ of each phase 40 of the second liquid in the flow part 172 is preferably within the same range as that of the length L₃₀ of the phase 30 of the first liquid described above. With this range, it is possible to achieve the same advantageous effect as explained for the length L₃₀ of the phase 30 of the first liquid.

The temperatures of the first liquid 10 and the second liquid 20 in the flow part 172 are not limited. From the viewpoint of facilitating the fast transfer of the object component from the first liquid 10 to the second liquid 20 to achieve fast extraction, the temperatures of the first liquid 10 and the second liquid 20 are preferably 10°C or higher, more preferably 20°C or higher, still more preferably 30°C or higher, and particularly preferably 40°C or higher. From the viewpoint of achieving more stable separation of the microorganism, the temperatures of the first liquid 10 and the second liquid 20 are preferably 90°C or lower, more preferably 80°C or lower, still more preferably 70°C or lower, particularly preferably 60°C or lower, particularly preferably 50°C or lower.

After the first liquid 10 and the second liquid 20 pass through the flow part 172 of the flow channel 170, the first liquid 10 and the second liquid 20 that have passed through the flow part 172 are discharged from the outlet part 173. Since the first liquid 10 and the second liquid 20 pass through the flow part 172 in a state where the phase of the first liquid 10 and the phase of the second liquid 20 are separated from each other, the phase of the first liquid 10 and the phase of the second liquid 20 are usually separated from each other even when the first liquid 10 and the second liquid 20 are discharged through the outlet part 173.

Afterward, the first liquid 10 and the second liquid 20 discharged from the outlet part 173 are collected into the collector 160 as illustrated in FIG. 1. The first liquid 10 and the second liquid 20 collected into the common collector 160 quickly cause phase separation. Thus, the phase of the first liquid 10 containing the first solvent and the microorganism as well as the phase of the second liquid 20 as the extraction liquid containing the second solvent and the object component are obtained in the collector 160. Since the phase separation occurs, it is easy to extract the phase of the second liquid 20.

In the collector 160, the second solvent may be contained in the phase of the first liquid 10. In such a case that the second solvent is contained in the phase of the first liquid 10, the amount of the phase of the second liquid 20 may be reduced by the amount, and the amount of the second liquid 20 obtained as the extraction liquid may be reduced. Therefore, in the extraction method described above, it is preferable to employ a condition that the second solvent is hardly contained in the phase of the first liquid 10.

The extraction ratio in the aforementioned extraction method is usually 10% or more, preferably 15% or more, more preferably 20% or more, still more preferably 40% or more, and particularly preferably 60% or more. The extraction ratio can be measured by the following method.

The concentration of the object component in the second liquid 20 collected in the collector 160 is measured. The concentration can be measured by gas chromatography. This concentration is multiplied by the amount of the second liquid 20 collected in the collector 160 to obtain the extraction amount of the object component. The extraction amount is divided by the amount of the object component contained in the first liquid before the extraction to obtain the extraction ratio.

As described above, according to the extraction method of the present embodiment, it is possible to simultaneously perform the separation of a microorganism and the extraction of the object component, in order to obtain the second liquid 20 that contains the object component without the microorganism as the extraction liquid. According to this extraction method, it is not necessary to separately perform a step of separating the microorganism from the first liquid 10 before the extraction step. Thus, the extraction method according to the present embodiment can be carried out in a short time. In the extraction method according to the present embodiment, it is possible to perform the separation of the microorganism and the extraction of the object component simultaneously, and thereby facilitate the operation by reducing the number of processes.

Furthermore, in the extraction method according to the present embodiment, it is possible to perform the extraction of the object component and the separation of the microorganism within a short time in which the first liquid 10 and the second liquid 20 pass through the flow part 172 of the flow channel 170. The time in which the first liquid 10 and the second liquid 20 pass through the flow part 172 refers to an extraction time in which the first liquid 10 and the second liquid 20 come into contact with each other for the extraction. In the extraction method according to the embodiment described above, the extraction time can be precisely controlled. For example, the extraction time can be precisely controlled by controlling factors such as the flow amount of the first liquid 10 supplied to the flow channel 170, the length of the flow part 172 of the flow channel 170.

The extraction method according to the aforementioned embodiment may be modified for the implementation.

For example, the extraction apparatus 100 described above may be provided with a first circulation portion (not illustrated) capable of returning the first liquid 10 collected in the collector 160 to the first container 110. This portion enables reuse of the first liquid 10 that is returned to the first container 110 after being collected in the collector 160, for the extraction of the object component. Thus, it is possible to increase the extraction ratio and reduce the amount of residual object component that is left in the first liquid 10 without being extracted.

For example, the extraction apparatus 100 described above may be provided with a second circulation portion (not illustrated) capable of returning the second liquid 20 collected in the collector 160 to the second container 130. This portion enables reuse of the second liquid 20 that is returned to the second container 130 after being collected in the collector 160, for the extraction of the object component. Thus, it is possible to reduce the amount of the second liquid 20 used for the extraction.

### [2. Second embodiment]

The extraction method described above can be applied to a method for producing the object component using a microorganism. In this production method, the microorganism is cultured in the first liquid as a culture medium to generate the object component, and then the generated object component is extracted with the second liquid to obtain the extraction liquid containing the object component. Hereinafter, one embodiment according to the method for producing the object component is described with reference to drawings.

FIG. 4 is a schematic diagram schematically illustrating a production apparatus 200 according to a second embodiment of the present invention. In FIG. 4, the same parts as for the extraction apparatus 100 explained for the first embodiment are designated by the same reference numerals as in the first embodiment.

As illustrated in FIG. 4, the production apparatus 200 according to the second embodiment of the present invention comprises the first container 110, the first supplying portion 120, the second container 130, the second supplying portion 140, the extraction duct 150 having the flow channel 170 (see FIG. 2) inside thereof, the collector 160, a first circulation portion 280, and a second circulation portion 290.

The first container 110, the first supplying portion 120, the second container 130, the second supplying portion 140, the extraction duct 150, the collector 160, and the flow channel 170 in the extraction duct 150 are the same as those described in the first embodiment.

It is desirable that the first container 110 is provided such that culturing conditions such as temperature and atmosphere can be set to appropriate ranges for the purpose of enabling the microorganism in the first liquid 10 stored in the first container 110 to generate the object component. The amounts of the microorganism and the object component of the first liquid 10 in the first container 110 may be changed depending on the culturing. Even in such a case, it is desired that the concentrations of the microorganism and the object component in the first liquid 10 to be supplied to the flow channel 170 is preferably within the range described for the first embodiment at a time of supplying the first liquid 10 to the flow channel 170 by the first supplying portion 120.

The first circulation portion 280 is a device provided in such a way as to be able to return the first liquid 10 collected in the collector 160 to the first container 110. In an example illustrated for this embodiment, the first circulation portion 280 comprises a first circulation duct 281 as a first circulation channel connected to the collector 160 and the first container 110, and a first circulation pump 282 as a first circulation amount adjustment portion provided in the first circulation duct 281. The first circulation portion 280 is provided such that the first liquid 10 collected in the collector 160 can be returned to the first container 110 through the first circulation duct 281. The first circulation portion 280 is provided such that the flow amount of the first liquid 10 returned through the first circulation duct 281 can be controlled by the first circulation pump 282.

The second circulation portion 290 is a device provided in such a way as to be able to return the second liquid 20 collected in the collector 160 to the second container 130. In an example illustrated for this embodiment, the second circulation portion 290 comprises a second circulation duct 291 as a second circulation channel connected to the collector 160 and the second container 130, and a second circulation pump 292 as a second circulation amount adjustment portion provided in the second circulation duct 291. The second circulation portion 290 is provided such that the second liquid 20 collected in the collector 160 can be returned to the second container 130 through the second circulation duct 291. The second circulation portion 290 is provided such that the flow amount of the second liquid 20 returned through the second circulation duct 291 can be controlled by the second circulation pump 292.

With use of the production apparatus 200 described above, it is possible to carry out the production method for producing the object component by culturing the microorganism. The production method includes:
(6) a step of generating the object component by the microorganism in the first container 110;
(7) a step of supplying the first liquid 10 from the first container 110 to the merging part 171 of the flow channel 170 in the extraction duct 150;
(8) a step of supplying the second liquid 20 to the merging part 171;
(9) a step of passing the first liquid 10 and the second liquid 20 supplied to the merging part 171 through the flow part 172, in a state where the phase of the first liquid 10 and the phase of the second liquid 20 are separated from each other;
(10) a step of discharging the first liquid 10 and the second liquid 20 having passed through the flow part 172, from the outlet part 173; and
(11) a step of collecting the first liquid 10 and the second liquid 20 that have been discharged from the outlet part 173, into a collector 160.

In the production method, the first container 110 stores therein the first liquid 10 as the culture medium containing the microorganisms and the first solvent. In the first container 110, the microorganism contained in the first liquid 10 is cultured. This culturing allows for the step of generating the object component by the microorganism to prepare the first liquid 10 that contains the microorganism, the object component and the first solvent.

Afterward, the step of supplying the first liquid 10 from the first container 110 to the merging part 171 of the flow channel 170 is performed. In the same way as in the first embodiment, the first liquid 10 is supplied to the merging part 171 through the first supplying duct 121 by using the first liquid feeding pump 122.

The step of supplying the second liquid 20 containing the second solvent from the second container 130 to the merging part 171 of the flow channel 170 is performed. In the same way as in the first embodiment, the second liquid 20 is supplied to the merging part 171 through the second supplying duct 141 by using the second liquid feeding pump 142.

Once the first liquid 10 and the second liquid 20 are supplied to the merging part 171, the first liquid 10 and the second liquid 20 merge. Then, while the phase of the first liquid 10 and the phase of the second liquid 20 are separated from each other, the step of passing the supplied first liquid 10 and the supplied second liquid 20 through the flow part 172 is performed. When the first liquid 10 and the second liquid 20 are passed through the flow part 172, the object component is transferred from the first liquid 10 to the second liquid 20 as in the first embodiment, while the microorganism is left in the phase of the first liquid 10. Thus, it is possible to proceed with the separation of the microorganism and the extraction of the object component simultaneously to obtain the second liquid 20 as the extraction liquid containing the second solvent and the object component. The passing conditions required for passing the first liquid 10 and the second liquid 20 through the flow part 172 are preferably controlled in the same way as in the first embodiment. With this control, it is possible to achieve the advantageous effects described in the first embodiment.

After the first liquid 10 and the second liquid 20 are passed through the flow part 172 of the flow channel 170, the step of discharging the first liquid 10 and the second liquid 20 passed through the flow part 172, from the outlet part 173, is performed as in the first embodiment.

Afterward, as in the first embodiment, the step of collecting the first liquid 10 and the second liquid 20 discharged from the outlet part 173 into the collector 160 is performed. The first liquid 10 and the second liquid 20 collected in the common collector 160 are separated quickly into the phase of the first liquid 10 containing the first solvent and the microorganism and the phase of the second liquid 20 containing the second solvent and the object component. Thus, it is possible to obtain the second liquid 20 that contains the object component without the microorganism from the phase of the second liquid 20 as the extraction liquid.

In this way, in the production method according to the present embodiment, after the object component is generated by the microorganism, it is possible to perform the separation of the microorganism and the extraction of the object component simultaneously, and thereby achieve an efficient production of the object component.

In the production method described above may include as required:
(12) a step of returning the first liquid 10 collected in the collector 160 to the first container 110.

In an example illustrated for this embodiment, the first circulation pump 282 is actuated to pump out the first liquid 10 from the collector 160. Then, the pumped first liquid 10 is returned to the first container 110 through the first circulation duct 281. The first liquid 10 returned to the first container 110 is used for culturing the microorganism in the first container 110. Afterward, the first liquid 10 is supplied again to the merging part 171 of the flow channel 170 through the first supplying portion 120. With such a step, it is possible to continuously perform the generation of the object component by the culturing of microorganism and the extraction of the object component. In the production method described above, the object component can be extracted within a short time, and thus it is possible to shorten the time required for circulating the first liquid 10 thorough the outside of the first container 110 functioning as a culture tank. Accordingly, it is possible to minimize loads on the microorganism resulting from oxygen deficiency.

When the extracting of the object component is carried out while the culturing of microorganism is continued, performing the aforementioned step of returning the first liquid 10 collected in the collector 160 to the first container 110 can make it possible to reduce the concentration of the object component in the first liquid 10 stored in the first container 110. The reduction of the concentration of the object component in the first liquid 10 in such a way is useful when the object component has a property of inhibiting the microorganism from generating the object component. For example, in the case that the object component has the property of inhibiting the microorganism from growing, the culturing of the microorganism may be stopped when the concentration of the object component in the first liquid 10 is increased to higher than the growth inhibition concentration. On the other hand, when the concentration of the object component in the first liquid 10 is maintained at the growth inhibiting concentration or lower, the continuous production of the object component is enabled.

Furthermore, the production method described above may include as required:
(13) a step of returning the second liquid 20 collected in the collector 160 to the second container 130.

In an example illustrated for this embodiment, the second circulation pump 292 is actuated to pump out the second liquid 20 from the collector 160. Then, the pumped second liquid 20 is returned to the second container 130 through the second circulation duct 291. The second liquid 20 returned to the second container 130 is supplied again to the merging part 171 of the flow channel 170 through the second supplying portion 140. With such a step, the second liquid 20 collected in the container 160 can be returned to the merging part 171 to be reused for the extraction of the object component. Thus, it is possible to reduce the amount of the second liquid 20 used for the extraction. Even in the case of a low extraction ratio with one time of passing through the channel 170, it is possible to improve the extraction ratio by increasing the number of times of passing through the flow channel 170 to twice or more.

As described above, in the production method according to the present embodiment, it is possible to perform the separation of the microorganism and the extraction of the object component simultaneously after the object component is generated by the culturing of the microorganism. As such, in this production method, the separation of the microorganism and the extraction of the object component do not need to be performed in different steps, and thus it is possible to perform the collection of the object component within a short time. The number of steps can be reduced, and thus it is possible to downscale the apparatus.

In conventional batch-type culture methods, the separation of the microorganism and extraction of the object component are generally carried out for the whole amount of the first liquid 10 after the culture is completed. Meanwhile, in the production method according to the present embodiment, the object component can be extracted from the first liquid 10 while the microorganism is cultured in the first container 110. Therefore, the production method according to the present embodiment does not require the process for the whole amount of the first liquid 10 at a time. Accordingly, the production method of the present embodiment enables downscaling of the facility.

In the production method according to the present embodiment, it is possible to precisely control the time for allowing the first liquid 10 to come into contact with the second liquid 20 in the flow channel 170 for the purpose of the extraction. Thus, it is possible to control the contact time between the first liquid 10 and the second liquid 20 in the flow channel 170 within a short time. In the production method according to the present embodiment, it is possible to quickly separate the phase of the first liquid 10 and the phase of the second liquid 20 that are collected in the collector 160. Thus, it is also possible to control the contact time between the first liquid 10 and the second liquid 20 in the collector 160 within a short time. Therefore, it is possible to minimize the time at which the component contained in the second liquid 20 comes into contact with the microorganism. Accordingly, any type of solvent harmful to the microorganism can be used as the second solvent. Consequently, in the producing method according to the present embodiment, it is possible to expand the range of choices for the solvent in the extraction solution.

The method for producing the object component according to the embodiment may be modified for the implementation.

For example, the first liquid 10 and the second liquid 20 may be added and extracted as necessary. In a specific example, after the extraction of the second liquid 20 collected in the collector 160, the second solvent and the object component may be separated from each other by a separation method such as distillation. The second solvent that is separated from the object component in such a way may be added to the second container 130 as the second liquid 20. With such a separation, solvent recycling of the second solvent can be carried out. Therefore, since the solvent can be repeatedly used, it is possible to reduce the amount of used solvent.

### [3. Object component]

The type of the object component is arbitrary. However, from the viewpoint of application to the production of the object component by the culturing of the microorganism as described in the second embodiment, the object component is preferably one capable of being generated by the microorganism.

Furthermore, since many types of microorganisms are cultured in a culture medium containing water, the object component is preferably a hydrophobic substance in order to smoothly perform the extraction. The hydrophobic substance refers to a substance that has a low solubility in water to such an extent as to allow the extraction by transferring the substance from the first liquid containing water to the second liquid. The solubility of the hydrophobic substance in water at normal temperature is preferably 10 g/kg or less, more preferably 5 g/kg or less, 1 g/kg or less, or 0.1 g/kg or less.

Examples of the hydrophobic substance include saturated hydrocarbons such as methane; unsaturated hydrocarbons such as ethylene, propylene, butadiene, isobutene and isoprene; aromatic compounds such as benzaldehyde, cinnamaldehyde, vanillin, eugenol, anethole and trimethylpyrazine; lipid compounds such as ceramide; lactone compounds such as nonalactone and decalactone; organic acid ester compounds such as isopentyl acetate, menthyl lactate, and monomenthyl succinate; and sugar alcohol compounds such as xylitol and arabitol.

In a preferred embodiment, the hydrophobic substance may be an isoprenoid compound. The isoprenoid compound includes one or more isoprene units having a molecular formula of (C₅H₈)ₙ. The precursor of the isoprene unit is isopentenyl pyrophosphate or dimethylallyl pyrophosphate. The isoprenoid compound is referred to also as terpene or terpenoid. The difference between terpene and terpenoid is that the terpene is a hydrocarbon while terpenoid contains more functional groups. Terpenes can be classified according to the number of isoprene units in the molecule [for example, hemiterpene (C5), monoterpene (C10), sesquiterpene (C15), diterpene (C20), sesterterpene (C25), triterpene (C30), sesquarterpene (C35), tetraterpene (C40), a polyterpene, and norisoprenoid]. Examples of monoterpene or monoterpenoid include pinene, nerol, citral, camphor, menthol, limonene, and linalool. Examples of the sesquiterpene or sesquiterpenoid includes nootkatone, valencene, nerolidol and farnesol. Examples of diterpene or diterpenoid includes phytol and vitamin A1. Squalene is an example of triterpene, and carotene (provitamin A1) is tetraterpene (Nature Chemical Biology 2,674-681 (2006); Nature Chemical Biology 5,283-291 (2009); Nature Reviews Microbiology 3,937-947 (2005); Adv Biochem Eng Biotechmol (DOI: 10.1007/10_2014_288).

In another preferred embodiment, the hydrophobic material may be a vanilloid compound. The vanilloid compound is a compound including a vanillyl group. Examples of the vanilloid compound include vanillin, vanillin acid, capsaicin, and vanillylmandelic acid.

### [4. Microorganism]

The type of microorganism is arbitrary. However, from the viewpoint of application to the production of the object component by culturing the microorganism as described in the second embodiment, it is preferable that the microorganism has the capability of generating the object component.

Examples of the microorganism include bacteria that are prokaryotic organisms and eumycetes that are eukaryotic organisms (e.g., yeast such as budding yeast and fission yeast). The bacteria may be gram-positive bacteria or gram-negative bacteria.

Examples of the gram-positive bacteria include Bacillus bacteria, Listeria bacteria, Staphylococcus bacteria, Streptococcus bacteria, Enterococcus bacteria, Clostridium bacteria, Corynebacterium bacteria and Streptomyces bacteria. Bacillus bacteria and Corynebacterium bacteria are preferred. Bacillus subtilis is preferred as Bacillus bacteria. Corynebacterium glutamicum is preferred as Corynebacterium bacteria.

Examples of the gram-negative bacteria include Escherichia bacteria, Pantoea bacteria, Salmonella bacteria, Vivrio bacteria, Serratia bacteria, and Enterobacter bacteria. Escherichia bacteria, Pantoea bacteria, and Enterobacter bacteria are preferred. Escherichia coli is preferred as the Escherichia bacteria. Pantoea ananatis is preferred as the Pantoea bacteria. Enterobacter aerogenes is preferred as the Enterobacter bacteria.

Examples of the eumycetes include Saccharomyces, Schizosaccharomyces, Yarrowia, Trichoderma, Aspergillus, Fusarium and Mucor. Microorganisms belonging to Saccharomyces, Schizosaccharomyces, Yarrowia, Trichoderma are preferred. As Saccharomyces, Saccharomyces cerevisiae is preferred. As Schizosaccharomyces, Schizosaccharomyces pombe is preferred. As Yarrowia, Yarrowia lipolytica is preferred. As Trichoderma, Trichoderma reesei is preferred.

The microorganism may be a genetically modified microorganism or a non-genetically modified microorganism. In order to obtain a large amount of the object component, genetically modified microorganism is preferable. Such a genetically modified microorganism has a modification of one or more genes involved in the generation of the object component. Examples of the genetically modified microorganism includes a microorganism having one or more heterologous gene involved in the generation of the object component (i.e., a gene that is not inherent to the microorganism), a microorganism having a modification (e.g., deletion, substitution, addition, insertion of one or more nucleotide residues in a coding region or a non-coding region of a genomic DNA) of one or more homogeneous gene involved in the generation of the object component (i.e., a gene inherent to the microorganism), and a microorganism that has one or more heterologous gene involved in the generation of the object component and has a modification of one or more homologous genes involved in the generation of the object component. Such a microorganism can be prepared by any known method in the relevant field. For example, preparation of the microorganism containing one or more homologous genes involved in the generation of the object component can be carried out by a method (e.g., competent cell method, electroporation method) using an expression vector (e.g., plasmid, virus vector, phage, artificial chromosome), and a genome editing technique (e.g., CRISPR/Cas system). In the case that the expression vector is an integrative vector that causes homologous recombination with genomic DNA of the microorganism, expression units containing the same type of gene and required elements (e.g., promoter) can be incorporated into the genomic DNA of the microorganism by transformation. On the other hand, in the case that the expression vector is a non-integrative vector that does not cause homologous recombination with genomic DNA of the microorganism, the expression units are not incorporated into the genomic DNA of the microorganism by transformation, and can be present as a state of expression vector in the microorganism independently from the genomic DNA. The preparation of microorganisms having modification of one or more homologous genes involved in the generation of the object component can be carried out by the genome editing technique, for example.

When the object component is an isoprenoid compound such as linalool, the microorganism is preferably a genetically modified microorganism containing a gene encoding one or more enzymes involved in biosynthesis of the isoprenoid compound. Pantoea ananatis SWITCH-PphoCΔgcd/AaLINS-ispA* strain (Patent application WO2017/051928) that is a genetically modified microorganism used in Examples has a gene encoding such an enzyme. Examples of one or more enzymes involved in biosynthesis of the isoprenoid compound include one or more enzymes involved in the MEP pathway, one or more enzymes involved in the MVA pathway, isopentenyl diphosphate Δ-isomerase, geranyl diphosphate synthase, farnesyl diphosphate synthase, and one or more enzymes involved in the biosynthesis of the object isoprenoid compound (e.g., linalool synthase, isoprene synthase, limonene synthase, myrcene synthase, amorphadiene synthase and farnesene synthase). Examples of one or more enzymes involved in the MEP pathway include 1-deoxy-D-xylulose-5-phosphate synthase, 1-deoxy-D-xylulose-5-phosphate reductoisomerase, 4-diphosphocytidyl-2-C-Methyl-D-erythritol synthase, 4-diphosphocytidyl-2-C-methyl-D-erythritol kinase, 2-C-methyl-D-erythritol-2,4-cyclodiphosphate synthase, 1-hydroxy-2-methyl-2-(E)-butenyl-4-diphosphate synthase and 4-hydroxy-3-methyl-2-butenyl diphosphate reductase (see, e.g., Kuzuyama T and Seto H, Proc Jpn Acad Ser B Phys Biol Sci 88, 41-52 (2012); Grawert T et al., Cell Mol Life Sci. 68, 3797-3814 (2011)) Examples of one or more enzymes involved in the MVA pathway include mevalonate kinase, phosphomevalonate kinase, diphosphomevalonate decarboxylase, Acetyl-CoA-C-acetyltransferase, hydroxymethyl glutaryl-CoA synthase, hydroxymethyl glutaryl-CoA reductase and acetyl-CoA-acetyltransferase/hydroxymethyl glutaryl-CoA reductase (see, e.g., Kuzuyama T and Seto H, Proc Jpn Acad Ser B Phys Biol Sci. 88, 41-52 (2012); Miziorko HM, Arch Biochem Biophys. 505,131-143 (2011))

When the object component is a vanilloid compound such as vanillin, the microorganism is preferably a genetically modified microorganism having a gene encoding one or more enzymes involved in biosynthesis of the vanilloid compound. Genetically modified Corynebacterium glutamicum used in Examples includes a gene encoding such an enzyme. Examples of one or more enzymes involved in the biosynthesis of vanilloid compounds include aromatic carboxylic acid reductase.

The generation of the object component by using the microorganism can be carried out by culturing the microorganism in an appropriate culture medium.

The culture medium is required to contain a component necessary for growth of the microorganism and the generation of the object component by the microorganism. As such, the culture medium preferably contains a carbon source and a nitrogen source. Examples of the carbon source include carbohydrates such as monosaccharides, disaccharides, oligosaccharides and polysaccharides; an invertose obtained by hydrolysis of sucrose; glycerol; methanol, formaldehyde, formate; oils such as corn oil, palm oil and soybean oil; acetates; animal fats; animal oils; fatty acids such as saturated fatty acids and unsaturated fatty acids; lipids; phospholipids; glycerolipids; glycerin fatty acid esters such as monoglycerides, diglycerides and triglyceride; polypeptides such as microorganism proteins or plant proteins; reproducible carbon sources such as hydrolyzed biomass carbon sources; yeast extract; or a combination thereof. Examples of the nitrogen source include inorganic ammonium salts such as ammonium sulfate, ammonium chloride, ammonium phosphate; organic nitrogen such as soybean hydrolysates; ammonia gas; and ammonia water. The culture medium may also contain an appropriate amount of required substances such as vitamin B1 and L-homoserine, or yeast extract as organic micronutrient sources. The culture medium may further contain an appropriate amount of potassium phosphate, magnesium sulfate, iron ions, and manganese ions as inorganic micronutrient sources.

Standard culture conditions can be used as culture conditions of the microorganism. The culture temperature may be 20-40°C and pH may be about 4.5 to about 9.5.

The culture may also be carried out under aerobic, non-oxygen, or anaerobic conditions depending on the type of microorganism. The culture period of the microorganism can be controlled as appropriate.

With the culture of microorganism in the culture medium as described above, it is possible to obtain the culture medium containing the microorganism and the object component as the first liquid. According to the method of the present invention, it is possible to efficiently extract and produce the object component from the culture medium containing the microorganism and the object component in addition to such a wide variety of components. With use of the medium containing no hydrophobic substance as the carbon source and the nitrogen source, the object component can be extracted or produced more efficiently from the culture medium.

### Examples

Hereinafter, the present invention is described in detail with reference to Examples. However, the present invention is not limited to the following Examples. The following operation was performed in a normal temperature and normal pressure in an atmospheric air, unless otherwise specified.

### [Production Example 1: Preparation of culture medium containing linalool]

A genetically modified microorganism, Pantoea ananatis SWITCH-PphoCΔgcd/AaLINS-ispA* strain (Patent application WO2017/051928) was cultured to generate linalool. Materials for media listed in Table 1 were used for the culture. The culture was carried out for 30 hours. The culture temperature was set to 34°C for the first 7 hours of the culture, and 30°C thereafter. Culture pH was controlled to 6.8 by using ammonia gas. Ventilation was carried out at 300 mL/minute. Dissolved oxygen concentration was set to 21% at the beginning of culture prior to inoculation. Dissolved oxygen concentration was set to 0% for saturated sodium sulfite solution. Stirring was controlled to maintain a dissolved oxygen concentration of 5% or more in the culture medium with use of a galvanic type sensor (ABLE Corporation).

### [Table 1]

**Table 1. Composition of Glucose media**

| Composition A | (concentration) |
|---|---|
| Glucose | 200 g/L |
| MgSO₄.7aq | 2.0 g/L |

| Composition B | |
|---|---|
| Trisodium citrate | 2.0 g/L |
| (NH₄)₂SO₄ | 2.0 g/L |
| KH₂PO₄ | 2.0 g/L |
| FeSO₄.7aq | 20 mg/L |
| MnSO₄.5aq | 20 mg/L |
| Yeast Extract | 4.0 g/L |
| DISFOAM GD-113K | 0.02 mL/L |

After 0.15 L of compositions were prepared for a composition A and a composition B (without pH adjustment), the compositions were sterilized by heat at 120°C for 20 minutes. After cooled down, the compositions for compositions A and B were mixed at 1:1. Then, kanamycin (100 mg/L) was added to the resulting mixture for use as a medium.

After the culture was initiated, samples were taken as appropriate for analysis of O.D. value and linalool. The linalool concentration was measured using a gas chromatograph GC-2025AF (manufactured by Shimadzu Corporation). The O.D. value was measured by a spectrophotometer (HITACHI U-2900) at 600 nm. The dry microorganism weight (g/L) of P. ananatis can be obtained by multiplying the value of O.D. 600 nm by 0.287. The linalool standard solution was prepared with a reagent linalool (manufactured by Wako Pure Chemical Corporation). The sample for the measurement was diluted with ethanol (manufactured by Wako Pure Chemical Corporation) as appropriate.

The culturing was completed after the culture for 30 minutes. The concentration of linalool in the culture medium at the end of culturing was 0.9 g/L, O.D. 600 nm was 23.5. That is, the dry microorganism weight was 6.74 g/L.

### [Production Example 2: Preparation of sterilized broth containing vanillin at pH 3.0]

A culture medium containing vanillic acid was used to culture Corynebacterium glutamicum with introduction of aromatic carboxylic acid reductase gene that derives from a genetically modified microorganism, Gordonia effusa in order to prepare a vanillin culture medium. Materials for media listed in Table 2 were used for the culture.

### [Table 2]

**Table 2. Composition of media**

| Composition A | |
|---|---|
| Media composition | Conc. |
| (A) VA broth | 14.2g/L |

| Composition B | |
|---|---|
| Media composition | Conc. |
| (B) | |
| H₃PO₄ | 2.00g/L |
| MgSO₄•7H₂O | 0.50g/L |
| FeSO₄•7H₂O | 40.00mg/L |
| MnSO₄•5H₂O | 40.00mg/L |
| KW Manero HCl(TN) | 0.600g/L |
| DISFOAM GD-113k | 0.30mL/L |

| pH 6.0 (KOH) | |
|---|---|
| VB1.HCL | 1.00mg/L |
| Biothin | 3.00mg/L |

| Composition C | |
|---|---|
| Glucose | 630 g/L |

The compositions for A, B, and C were sterilized, and then mixed at a ratio of 80.0:5.7:4.5 for the preparation of medium. The pH was 7.2.

The solution of composition C was used as appropriate for feeding and culturing to obtain a culture medium containing vanillin. The vanillin concentration in the culture medium at the end of culturing was 9.4 g/L, O.D. 562 nm was 3.15. The dry microorganism weight of the Corynebacterium glutamicum can be obtained by multiplying O.D. 562 nm by 30.1. As a result, the dry microorganism weight at this time was 94.9 g/L.

The pH of the culture medium was adjusted to 3.0 using 98% sulfuric acid. Then, the medium was heated to about 80°C for sterilization to obtain a sterilized broth having a pH of 3.0 containing vanillin. The vanillin concentration in the resulting sterilized broth was 9.3 g/L. O.D. 562 nm was 31.0. The dry microorganism weight was 93 g/L.

### [Production Example 3: Preparation of sterilized broth of pH 6.0 containing vanillin]

The pH of the sterilized broth prepared in Production Example 2 was adjusted to 6.0 using a NaOH aqueous solution having a concentration of 29% to obtain a sterilized broth with pH 6.0 containing vanillin. The vanillin concentration in the resulting sterilization broth was 9.1 g/L, O.D. 562 nm was 3.04. The dry microorganism weight was 91.5 g/L.

### [Production Example 4: Preparation of sterilized broth of pH 3.0 containing vanillin]

The culture medium containing vanillic acid was used to culture Corynebacterium glutamicum with introduction of aromatic carboxylic acid reductase gene that derives from a genetically modified microorganism, Gordonia effusa in order to prepare the vanillin culture medium. Materials for media listed in Table 3 were used for the culture.

### [Table 3]

**Table 3. Composition of Media**

| Composition A | |
|---|---|
| Media composition | Conc. |
| (A) VA broth | 14.9g/L |

| Composition B | |
|---|---|
| Media composition | Conc. |
| (B) | |
| H₃PO₄ | 2.00g/L |
| MgSO₄•7H₂O | 0.50g/L |
| FeSO₄•7H₂O | 40.00mg/L |
| MnSO₄•5H₂O | 40.00mg/L |
| KW Manero HCl(TN) | 0.600g/L |
| DISFOAM GD-113k | 0.30mL/L |

| pH 6.0(KOH) | |
|---|---|
| VB1.HCL | 1.00mg/L |
| Biothin | 3.00mg/L |

| Composition C | |
|---|---|
| Glucose | 640 g/L |

The compositions for A, B, and C were sterilized, and then mixed at a ratio of 80.8:6.3:4.6 for the preparation of medium. The pH was 7.2.

The solution of composition C was used as appropriate for feeding and culturing to obtain the culture medium containing vanillin. The vanillin concentration in the culture medium at the end of culturing was 10.0 g/L, O.D. 562 nm was 3.11. The dry microorganism weight of the Corynebacterium glutamicum can be obtained by multiplying O.D. 562 nm by 30.1. As a result, the dry microorganism weight at this time was 93.8 g/L.

The pH of the culture medium was adjusted to 3.0 using 98% sulfuric acid. Then, the medium was heated to about 80°C for sterilization to obtain a sterilized broth having a pH of 3.0 containing vanillin. The vanillin concentration in the resulting sterilized broth was 9.8 g/L. O.D. 562 nm was 3.05. The dry microorganism weight was 92 g/L.

### [Production Example 5: Preparation of sterilized broth of pH 6.0 containing vanillin]

The pH of the sterilized broth prepared in Production Example 4 was adjusted to 6.0 using a NaOH aqueous solution having a concentration of 29% to obtain a sterilized broth with pH 6.0 containing vanillin. The vanillin concentration in the resulting sterilization broth was 9.6 g/L, O.D. 562 nm was 2.99. The dry microorganism weight was 90 g/L.

### [Examples 1-9]

### (Preparation of flow channel)

A T-shaped micromixer having a first supplying port (inner diameter 1.59 mm), a second supplying port (inner diameter 1.59 mm) and an outlet port (inner diameter 2.18 mm) was prepared. A stainless tube (formed of SUS316, inner diameter 2.18 mm) having a length listed in Table 4 was prepared. The tube was connected to the outlet port of the micromixer to prepare an extraction duct 150 having a flow channel 170 therein as illustrated in FIG. 2. In the flow channel 170 in the extraction duct 150, a merging part 171 and a flow part 172 correspond to a part inside the T-shaped micromixer and a part inside the tube, respectively. The flow part 172 was shaped into a cylinder with a constant inner diameter.

### (Extraction operation)

A constant amount of the culture medium prepared in Production Example 1 was continuously supplied as a first liquid to the first supplying port of the micromixer. At the same time, a constant amount of isopropyl myristate was continuously supplied as a second liquid to the second supplying port of the micromixer. The supplied culture medium and isopropyl myristate merged at the merging part inside the micromixer, passed through the flow part inside the tube, and were flowed into a collector. The first liquid and the second liquid flowed into the collector were quickly separated into a phase of the first liquid and a phase of the second liquid. Since the quick phase separation was achieved in the collector in this way, it is assured that the phase of the first liquid and the phase of the second liquid were separated from each other in the flow part inside the tube. Since Examples 10 to 23, in which close flow amounts were employed as described below, were confirmed to cause slug flows, the inventors determined that the slug flows were formed in the flow part inside the tube also in Examples 1 to 9. In the above operation, the temperature of the flow channel is set to normal temperature. The experiment was carried out under an atmosphere having normal pressure.

### [Examples 10-23]

### (Preparation of flow channel)

The T-shaped micromixer having the first supplying port (inner diameter 1.59 mm), the second supplying port (inner diameter 1.59 mm) and the outlet port (inner diameter 1.59 mm) was prepared. A tetrafluoroethylene-perfluoroalkyl vinyl ether copolymer tube (formed of PFA, inner diameter 1.59 mm) having a length listed in Table 4 was prepared. The tube was connected to the outlet port of the micromixer to prepare the extraction duct 150 having the flow channel 170 therein as illustrated in FIG. 2. In the flow channel 170 in the extraction duct 150, the merging part 171 and the flow part 172 correspond to the part inside the T-shaped micromixer and the part inside the tube, respectively. The flow part 172 was shaped into a cylinder with a constant inner diameter.

A constant amount of the sterilized broth with pH 3.0 prepared in Production Example 2 was continuously supplied as the first liquid to the first supplying port of the micromixer. At the same time, a constant amount of toluene was continuously supplied as the second liquid to the second supplying port of the micromixer. The supplied sterilized broth and toluene merged at the merging part inside the micromixer, passed through the flow part inside the tube, and were flowed into the collector. The slug flow was confirmed by visual observation of the flows of the first liquid and the second liquid that were passing through the flow part inside the tube. The first liquid and the second liquid flowed into the collector were quickly separated into the phase of the first liquid and the phase of the second liquid. In the above operation, the temperature of the flow channel was set to normal temperature. The experiment was carried out under an atmosphere having normal pressure.

### [Examples 24-38]

### (Preparation of flow channel)

The T-shaped micromixer having the first supplying port (inner diameter 1.59 mm), the second supplying port (inner diameter 1.59 mm) and the outlet port (inner diameter 2.18 mm) was prepared. A stainless tube (formed of SUS316, inner diameter 2.18 mm) having a length listed in Table 4 was prepared. The tube was connected to the outlet port of the micromixer to prepare the extraction duct 150 having the flow channel 170 therein as illustrated in FIG. 2. In the flow channel 170 in the extraction duct 150, the merging part 171 and the flow part 172 correspond to the part inside the T-shaped micromixer and the part inside the tube, respectively. The flow part 172 was shaped into a cylinder with a constant inner diameter.

A constant amount of the sterilized broth with pH 6.0 prepared in Production Example 3 was continuously supplied as the first liquid to the first supplying port of the micromixer. At the same time, a constant amount of toluene was continuously supplied as the second liquid to the second supplying port of the micromixer. The supplied sterilized broth and toluene merged at the merging part inside the micromixer, passed through the flow part inside the tube, and were flowed into the collector. The first liquid and the second liquid flowed into the collector were quickly separated into the phase of the first liquid and the phase of the second liquid. Since the quick phase separation was achieved in the collector in this way, it is assured that the phase of the first liquid and the phase of the second liquid were separated from each other in the flow part in the tube. Since Examples 10 to 23, in which close flow amounts were employed as described above, were confirmed to cause slug flows, the inventors determined that the slug flows are formed in the flow part inside the tube also in Examples 24 to 38. In the above operation, the temperature of the flow channel is set to normal temperature. The experiment was carried out under an atmosphere having normal pressure.

### [Examples 39-73]

### (Preparation of flow channel)

The T-shaped micromixer having the first supplying port (inner diameter 6.35 mm), the second supplying port (inner diameter 6.35 mm) and the outlet port (inner diameter 6.35 mm) was prepared. The stainless tube (formed of SUS316, inner diameter 6.35 mm) having a length listed in Table 5 was prepared. The tube was connected to the outlet port of the micromixer to prepare the extraction duct 150 having the flow channel 170 therein as illustrated in FIG. 2. In the flow channel 170 in the extraction duct 150, the merging part 171 and the flow part 172 correspond to the part inside the T-shaped micromixer and the part inside the tube, respectively. The flow part 172 was shaped into a cylinder with a constant inner diameter.

A liquid supplying duct that comprised a heating duct (formed of SUS 316, inner diameter 2.18 mm) capable of heating a liquid flowing therein and a temperature measuring portion capable of directly measuring a temperature of the heated liquid inside the duct, was provided. The liquid supplying ducts were connected to the first supplying port and the second supplying port of the micromixer. Then, whole parts of the micromixer and the tube as the flow part were dipped into a thermostatic bath except the vicinity of the outlet of the tube. Temperatures of the heating duct and the thermostatic bath were set such that the liquid temperatures can be controlled at extraction temperatures listed in Table 5 for the first liquid and the second liquid between the supplying to the micromixer and flowing into the collector.

The first liquid and the second liquid flowed under the controlled temperature. Specifically, a constant amount of the sterilized broth with pH 6.0 prepared in Production Example 5 was continuously supplied as the first liquid to the first supplying port of the micromixer. At the same time, a constant amount of toluene was continuously supplied as the second liquid to the second supplying port of the micromixer. The supplied sterilized broth and toluene merged at the merging part inside the micromixer, passed through the flow part inside the tube, and were flowed into the collector. The first liquid and the second liquid flowed into the collector were quickly separated into the phase of the first liquid and the phase of the second liquid. Since the quick phase separation was achieved in the collector in this way, it is assured that the phase of the first liquid and the phase of the second liquid were separated from each other in the flow part in the tube. Since Examples 10 to 23, in which close flow amounts were employed as described above, were confirmed to cause slug flows, the inventors determined that the slug flows are formed in the flow part inside the tube also in Examples 39 to 73.

### [Comparative Examples 1 and 2]

The same operations were carried out in the same manner as in Examples 10 to 23 except that the extract conditions of the sterilized broth and toluene were modified as listed in Table 6. In Comparative Examples 1 and 2, the phase separation of the first liquid and the second liquid in the collector was not recognized. Since the phase separation was not recognized in the collector, the inventors determined that, in Comparative Examples 1 and 2, the first liquid and the second liquid did not cause the phase separation in the flow part inside the tube.

### [Comparative Example 3]

The same operations were carried out in the same manner as in Examples 24 to 38 except that the extract conditions of the sterilized broth and toluene were modified as listed in Table 6. In Comparative Example 3, the phase separation of the first liquid and the second liquid in the collector was not recognized. Since the phase separation was not recognized in the collector, the inventors determined that, in Comparative Example 3, the first liquid and the second liquid did not cause the phase separation in the flow part inside the tube.

### [Comparative Example 4]

4 mL of the linalool containing-culture medium produced in Production Example 1 and 4 mL of isopropyl myristate were added into a polypropylene tube container with a 15 mL capacity, and then the tube was sealed hermetically. Afterwards, the tube was shaken vigorously by hand for stirring the mixture.

### [Comparative Examples 5-8]

3 mL of the containing-sterilized broth with pH 3.0 produced in Production Example 2 and 3 mL of the second liquid listed in Table 6 were added into the polypropylene tube container with a 15 mL capacity, and then the tube was sealed hermetically. Afterwards, the tube was shaken vigorously by hand for stirring the mixture.

### [Comparative Examples 9-12]

3 mL of the vanillin containing-sterilized broth with pH 6.0 produced in Production Example 3 and 3 mL of the second liquid listed in Table 6 were added into the polypropylene tube container with a 15 mL capacity, and then the tube was sealed hermetically. Afterwards, the tube was shaken vigorously by hand for stirring the mixture.

### [Evaluation of separability in Examples 1 to 73 and Comparative Examples 1 to 12]

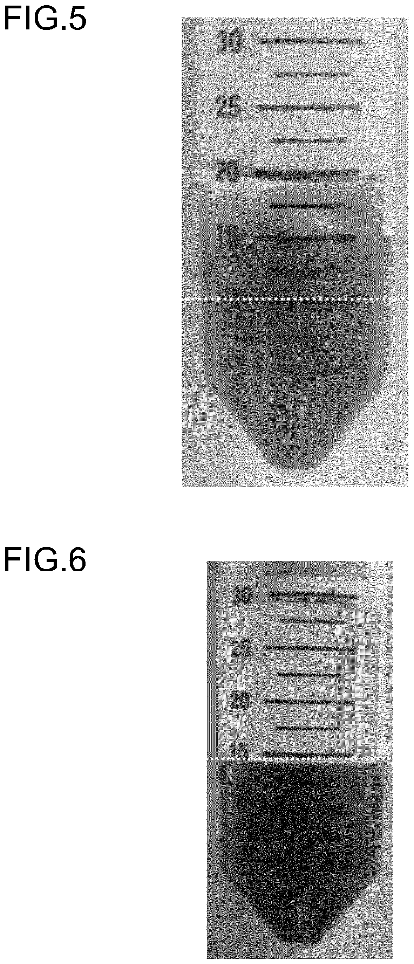

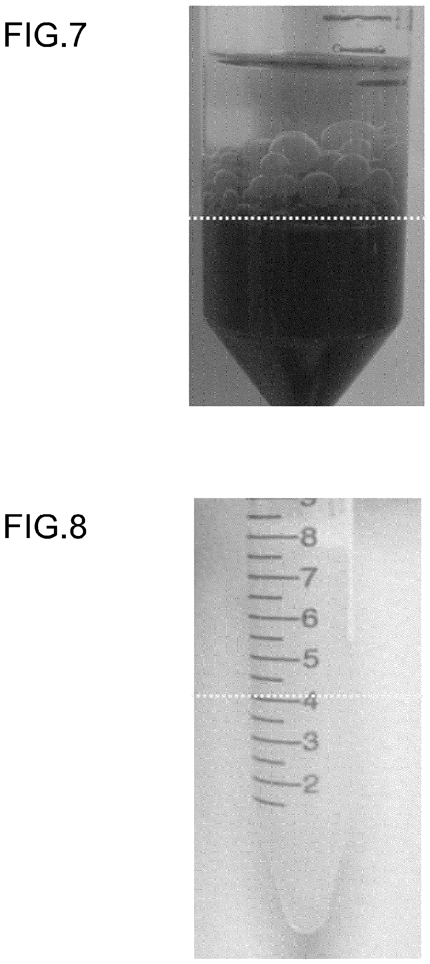

In Examples 1 to 73 and Comparative Examples 1 to 3, the state of the separation between the first liquid (culture medium or sterilized broth) and the second liquid (isopropyl myristate or toluene) was visually observed immediately after the first liquid and the second liquid are flowed into the collector. The observation results are evaluated according to the following criteria:
"5": Quick separation between the phase of the first liquid and the phase of the second liquid (See FIG. 6);
"4": Quick separation between the phase of the first liquid and the phase of the second liquid, in which the phase of the first liquid contains a part of the second liquid (See FIG. 7);
"3": Quick separation between the phase of the first liquid and the phase of the second liquid, in which the phase of the first liquid contains a large part of the second liquid (See FIG. 5);
"2": Separation between the phase of the first liquid and the phase of the second liquid after the lapse of time; and
"1": No phase separation between the phase of the first liquid and the phase of the second liquid.

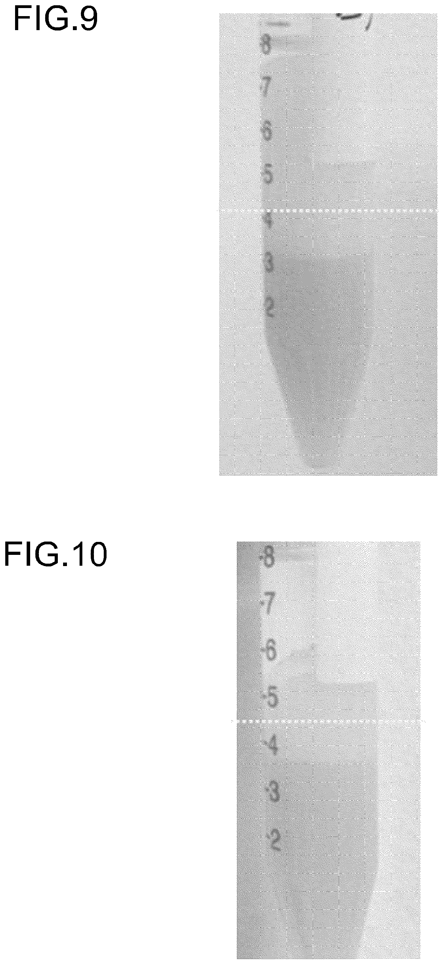

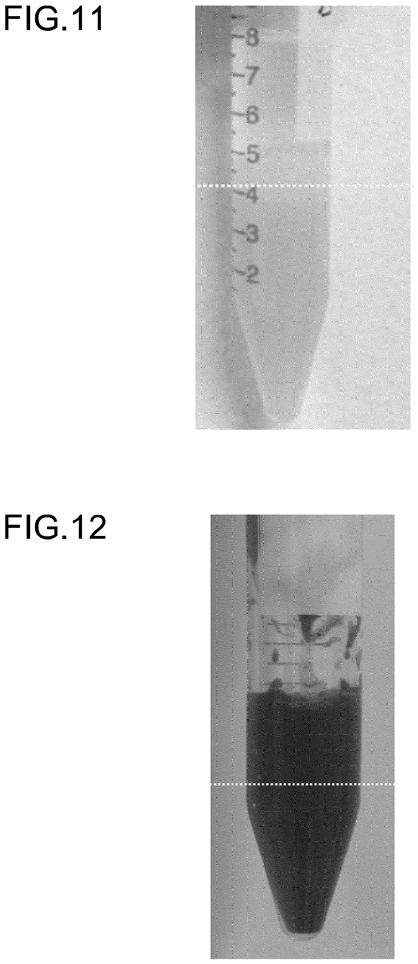

No phase separation was observed in Comparative Examples 4 to 12, between the phase of the first liquid and the phase of the second liquid immediately after the stirring. Then, the tube is left to stand for continuous observation. The observation results were determined according to the following criteria:
"3'": Separation between the phase of the first liquid and the phase of the second liquid after the container was left to stand for several minutes, in which the phase of the first liquid contains a large part of the second liquid;
"2'": Separation between the phase of the first liquid and the phase of the second liquid after the lapse of time (See FIGS. 8 to 11); and
"1'": No phase separation between the phase of the first liquid and the phase of the second liquid (See FIG. 12) .

### [Measurement of extraction ratios in Examples 1-73 and Comparative Examples 1-12]

The second liquid was sampled from the collector or the tube container. The concentration of the object component (linalool or vanillin) contained in the second liquid was measured by gas chromatography. The measured concentration was multiplied by the flow amount of the second liquid to obtain the amount of the object component transferred to the second liquid. The obtained amount was quantified as the extraction amount. On the other hand, the concentration of the object component in the original solution to be used for the extraction (the culture medium containing linalool, the sterilized broth with pH 3.0 containing vanillin, or the sterilized broth with pH 6.0 containing vanillin) was multiplied by the flow amount of the original solution to obtain the total amount of the object component contained in the original solution. Then, the extraction amount was divided by the total amount of the object component to obtain the extraction ratio.

### [Results of Examples 1-73 and Comparative Examples 1-12]

Results of Examples 1 to 73 and Comparative Examples 1 to 12 are shown in the following Tables 4 to 6. In the case of 15% or more of the extraction rate with the separability evaluated as "3" to "5", it was determined that the problem of simultaneously performing the separation of microorganism and the extraction of object component can be solved.

In Table 6, since Comparative Examples 4 to 12 are based on batch methods, values in the column of the supply amount represent the amount [mL] added into the tube, and the flowing time represents the stirring time. In the following tables, the abbreviations represent as follows:
IPM: Isopropyl myristate
Tr: Toluene
SUS: Stainless steel
PFA: Tetrafluoroethylene-perfluoroalkyl vinyl ether copolymer
PP: Polypropylene

### [Table 4]

**Table 4. Results of Examples 1 - 38]**

| | | | | | | | | | | |
|---|---|---|---|---|---|---|---|---|---|---|
| | first liquid | | second liquid | | flow part | | | | result | |

| Ex. | component | supplying amount | solvent | supplying amount | inner diameter | length | flowing time | material | separability | extraction ratio |
|---|---|---|---|---|---|---|---|---|---|---|
| | | mL/min. | | mL/min. | mm | m | sec. | | | % |
| 1 | linalool | 20 | IPM | 20 | 2.18 | 8 | 45 | SUS | 5 | 89 |
| 2 | | 40 | IPM | 40 | | 8 | 23 | | 5 | 86 |
| 3 | | 59 | IPM | 59 | | 8 | 15 | | 5 | 93 |
| 4 | | 20 | IPM | 20 | | 2 | 11 | | 5 | 34 |
| 5 | | 40 | IPM | 40 | | 2 | 6 | | 5 | 42 |
| 6 | | 59 | IPM | 59 | | 2 | 4 | | 5 | 55 |
| 7 | | 20 | IPM | 20 | | 1 | 6 | | 5 | 26 |
| 8 | | 40 | IPM | 40 | | 1 | 3 | | 5 | 34 |
| 9 | | 59 | IPM | 59 | | 1 | 2 | | 5 | 47 |
| 10 | vanillin pH3.0 | 1 | Tr | 1 | 1.59 | 1 | 60 | PFA | 5 | 37 |
| 11 | | 2.5 | Tr | 2.5 | | 1 | 24 | | 5 | 28 |
| 12 | | 5 | Tr | 5 | | 1 | 12 | | 5 | 22 |
| 13 | | 10 | Tr | 10 | | 1 | 6 | | 5 | 18 |
| 14 | | 20 | Tr | 20 | | 1 | 3 | | 5 | 28 |
| 15 | | 30 | Tr | 30 | | 1 | 2 | | 5 | 30 |
| 16 | | 50 | Tr | 50 | | 1 | 1 | | 5 | 29 |
| 17 | | 100 | Tr | 100 | | 1 | 1 | | 5 | 26 |
| 18 | | 10 | Tr | 10 | | 8 | 48 | | 5 | 65 |
| 19 | | 10 | Tr | 10 | | 2 | 12 | | 5 | 40 |
| 20 | | 30 | Tr | 30 | | 2 | 4 | | 5 | 37 |
| 21 | | 50 | Tr | 50 | | 2 | 2 | | 5 | 34 |
| 22 | | 5 | Tr | 5 | | 8 | 95 | | 5 | 40 |
| 23 | | 2.5 | Tr | 2.5 | | 8 | 191 | | 5 | 55 |
| 24 | vanillin pH6.0 | 5 | Tr | 5 | 2.18 | 1 | 22 | SUS | 5 | 55 |
| 25 | | 9.4 | Tr | 9.4 | | 1 | 12 | | 5 | 53 |
| 26 | | 18.8 | Tr | 18.8 | | 1 | 6 | | 5 | 46 |
| 27 | | 39.2 | Tr | 39.2 | | 1 | 3 | | 5 | 44 |
| 28 | | 56.5 | Tr | 56.5 | | 1 | 2 | | 5 | 42 |
| 29 | | 5 | Tr | 5 | | 5 | 112 | | 5 | 66 |
| 30 | | 9.4 | Tr | 9.4 | | 5 | 60 | | 4 | 67 |
| 31 | | 18.8 | Tr | 18.8 | | 5 | 30 | | 4 | 68 |
| 32 | | 39.2 | Tr | 39.2 | | 5 | 14 | | 4 | 70 |
| 33 | | 56.5 | Tr | 56.5 | | 5 | 10 | | 4 | 68 |
| 34 | | 5 | Tr | 5 | | 10 | 224 | | 5 | 72 |
| 35 | | 9.4 | Tr | 9.4 | | 10 | 119 | | 3 | 74 |
| 36 | | 18.8 | Tr | 18.8 | | 10 | 60 | | 3 | 74 |
| 37 | | 39.2 | Tr | 39.2 | | 10 | 29 | | 3 | 76 |
| 38 | | 25.1 | Tr | 12.55 | | 10 | 59 | | 3 | 51 |

### [Table 5]

**[Table 5. Results on Examples 39 - 73]**

| Ex. | first liquid | | second liquid | | flow part | | | | extraction temp. | result | |
|---|---|---|---|---|---|---|---|---|---|---|---|
| | component | supplying amount | solvent | supplying amount | inner diameter | length | flowing time | material | | separab ility | extraction ratio |
| | | mL/min. | | mL/min. | mm | m | sec. | | °C | | % |
| 39 | vanillin pH6.0 | 10 | Tr | 10 | 6.35 | 1.7 | 159 | SUS | normal temp. | 5 | 35 |
| 40 | | 20 | | 20 | | | 79 | | normal temp. | 5 | 29 |
| 41 | | 42 | | 42 | | | 38 | | normal temp. | 5 | 22 |
| 42 | | 61 | | 61 | | | 26 | | normal temp. | 5 | 18 |
| 43 | | 79 | | 79 | | | 20 | | normal temp. | 5 | 15 |
| 44 | | 10 | | 10 | | 5.1 | 477 | | normal temp. | 5 | 58 |
| 45 | | 20 | | 20 | | | 238 | | normal temp. | 5 | 50 |
| 46 | | 42 | | 42 | | | 114 | | normal temp. | 5 | 40 |
| 47 | | 61 | | 61 | | | 78 | | normal temp. | 5 | 16 |
| 48 | | 79 | | 79 | | | 60 | | normal temp. | 5 | 15 |
| 49 | | 10 | | 10 | | 8.5 | 795 | | normal temp. | 5 | 73 |
| 50 | | 20 | | 20 | | | 397 | | normal temp. | 5 | 63 |
| 51 | | 42 | | 42 | | | 189 | | normal temp. | 5 | 54 |
| 52 | | 61 | | 61 | | | 130 | | normal temp. | 5 | 47 |
| 53 | | 79 | | 79 | | | 101 | | normal temp. | 5 | 22 |
| 54 | | 10 | | 10 | | 1.7 | 159 | | 40 | 5 | 44 |
| 55 | | 20 | | 20 | | | 79 | | 40 | 5 | 44 |
| 56 | | 42 | | 42 | | | 38 | | 40 | 5 | 31 |
| 57 | | 61 | | 61 | | | 26 | | 40 | 5 | 24 |
| 58 | | 79 | | 79 | | | 20 | | 40 | 5 | 21 |
| 59 | | 10 | | 10 | | 1.7 | 159 | | 75 | 3 | 55 |
| 60 | | 20 | | 20 | | | 79 | | 75 | 4 | 48 |
| 61 | | 42 | | 42 | | | 38 | | 75 | 4 | 38 |
| 62 | | 61 | | 61 | | | 26 | | 75 | 4 | 16 |
| 63 | | 79 | | 79 | | | 20 | | 75 | 3 | 15 |
| 64 | | 20 | | 20 | | 8.5 | 397 | | 40 | 5 | 79 |
| 65 | | 42 | | 42 | | | 189 | | 40 | 5 | 72 |
| 66 | | 61 | | 61 | | | 130 | | 40 | 5 | 67 |
| 67 | | 79 | | 79 | | | 101 | | 40 | 5 | 61 |
| 68 | | 10 | | 10 | | 1.7 | 159 | | 50 | 4 | 55 |
| 69 | | 20 | | 20 | | | 79 | | 50 | 4 | 50 |
| 70 | | 42 | | 42 | | | 38 | | 50 | 4 | 41 |
| 71 | | 10 | | 10 | | | 159 | | 60 | 4 | 64 |
| 72 | | 20 | | 20 | | | 79 | | 60 | 4 | 53 |
| 73 | | 42 | | 42 | | | 38 | | 60 | 4 | 46 |

### [Table 6]

**[Table 6. Results of Comparative Examples 1 - 12]**

| Comp. Ex | first liquid | | second liquid | | flow part | | | | result | |
|---|---|---|---|---|---|---|---|---|---|---|
| | component | supplying amount | solvent | supplying amount | inner diameter | length | flowing time | material | separab ility | extraction ratio |
| | | mL/min. | | mL/min. | mm | m | sec. | | | % |
| 1 | vanillin pH3.0 | 30 | Tr | 30 | 1.59 | 8 | 16 | PFA | 1 | not measurable |
| 2 | | 50 | Tr | 50 | | 8 | 10 | | 1 | not measurable |
| 3 | vanillin pH6.0 | 56.5 | Tr | 56.5 | 2.18 | 10 | 20 | SUS | 1 | not measurable |
| 4 | linalool | 4 | IPM | 4 | - | - | 60 | PP | 2 | 98 |
| 5 | vanillin pH3.0 | 3 | toluene | 3 | - | - | 60 | | 1 | not measurable |
| 6 | | 3 | IPM | 3 | - | - | 60 | | 1 | not measurable |
| 7 | | 3 | palm oil | 3 | - | - | 60 | | 1 | not measurable |
| 8 | | 3 | heptane | 3 | - | - | 60 | | 1 | not measurable |
| 9 | vanillin pH6.0 | 3 | toluene | 3 | - | - | 60 | | 3' | 73 |
| 10 | | 3 | IPM | 3 | - | - | 60 | | 1 | not measurable |
| 11 | | 3 | palm oil | 3 | - | - | 60 | | 1 | not measurable |
| 12 | | 3 | heptane | 3 | - | - | 60 | | 1 | not measurable |

FIGS. 5, 6 and 7 are photographs of the collector taken immediately after the first liquid and the second liquid were collected in Examples 59, 65 and 73.

FIGS. 8, 9, 10 and 11 are photographs of the tube container taken immediately, 20 minutes, 1 hour and 24 hours after the stirring was completed in Comparative Example 4, respectively.

FIG. 12 is a photograph of the tube container taken 60 hours after the stirring was completed in Comparative Example 5.

In FIGS. 5 to 12, broken lines represent positions of the interface between the phase of the first liquid and the phase of the second liquid on the assumption that there is no mixture between the first liquid and the second liquid.

As listed in Table 6, in the extraction operation based on batch method as in Comparative Examples 4 to 12, stirring may cause suspension of the first liquid and the second liquid to generate emulsification. In such a case of emulsification, the first liquid and the second liquid are often unable to be separated from each other even after being left to stand because of stabilized emulsification state. In order to separate, it is required to leave the liquids to stand for a long time, in general.

Meanwhile, as listed in Tables 4 and 5, Examples 1 to 73 achieve the extraction of the object components (linalool and vanillin) that are transferred to the second liquid from the first liquid by the passing through the flow path. In examples 1 to 73, the first liquid and the second liquid collected in the collector cause phase separation within a short time of several seconds. In all Examples, the phase of the second liquid was obtained as a supernatant phase generated by the phase separation. As illustrated in FIGS. 5 to 7, the supernatant phase contains no microorganisms.

The aforementioned results demonstrate that the present invention enables simultaneous implementation of the extraction of the object component and the separation of microorganism even when it is difficult to perform the extraction of the object component and the separation of microorganism in the extraction operation based on the batch method.

### [Example 74]

### (74-1. Fermentation condition)

Jar culture was carried out using Pantoea ananatis SWITCH-PphoCΔgcd/AaLINS-ispA* strain (WO2017/051928). For the jar culture, a jar fermentor (ABLE Corporation) was used as a fermentation tank with a 1 L capacity. Glucose medium was prepared according to the composition listed in Table 7.

The strain was applied on an LB plate containing kanamycin (50 mg/L) and then cultured at 34°C for 16 hours as a pre-culture. Next, 300 mL of the glucose medium (Table 7) described below was put into the jar fermentor with a 1 L capacity, and then one plate of sufficiently grown bacterial cells were inoculated for starting the culture. The culture temperature was set at 34°C for the first 11 hours, and 30°C afterward. pH for the culture was controlled to 6.8 with use of ammonia gas. Ventilation was carried out at 300 mL/minute. Dissolved oxygen concentration was set to 21% at the beginning of the culture prior to inoculation. Dissolved oxygen concentration was set to 0% for saturated sodium sulfite solution. Stirring was controlled to maintain a dissolved oxygen concentration of 5% or more in the culture medium with use of the galvanic type sensor (ABLE Corporation). The culture was carried out for 39.5 hours while a feed medium listed in Table 8 was added at 1.7 mL per hour to maintain the glucose concentration in a range of 10 to 50 g/L in the medium.

### [Table 7]

**Table 7. Composition of Glucose Media**

| Composition A | (concentration) |
|---|---|
| Glucose | 120 g/L |
| MgSO₄.7aq | 2.2 g/L |

| Composition B | |
|---|---|
| Trisodium citrate | 2.0g/L |
| (NH₄)₂SO₄ | 2.0g/L |
| KH₂PO₄ | 3.6g/L |
| Yeast Extract | 4.0g/L |
| FeSO₄.7aq | 20mg/L |
| MnSO₄.5aq | 20mg/L |
| DISFOAM GD-113K | 0.02 mL/L |

After 0.15 L of compositions were prepared for a composition A and a composition B (without pH adjustment), the compositions were sterilized by heat at 120°C for 20 minutes. After cooled down, the compositions for compositions A and B were mixed at 1:1. Then, kanamycin (100 mg/L in final concentration) was added to the mixture.

### [Table 8]

**Table 8. Composition of Feed Media**

| Feed Media | (concentration) |
|---|---|
| Glucose | 700 g/L |
| DISFOAM GD-113K | 0.7 mL/L |

After a 1 L medium was prepared (without pH adjustment), the medium was sterilized by heat at 120°C for 20 minutes.

After the culture was initiated, samples were taken as appropriate for GC analysis of linalool. The standard solution of linalool was prepared from the reagent linalool (manufactured by Wako Pure Chemical Corporation). The sample to be used for the measurement was diluted with ethanol (manufactured by Wako Pure Chemical Corporation) as appropriate.

GC analysis was carried out using HP-INNOWAX column under an inlet temperature of 250°C and a detector temperature of 250°C and a split ratio of 10. The column was heated up at 5°C/minute from 50°C, and the temperature was maintained at 230°C for 4 minutes. The linalool concentration was quantified according to a calibration curve that was prepared from a linalool standard solution with 100% ethanol. Each sample was diluted with 100% ethanol as appropriate to have a concentration within the concentration range of the standard solution.

### (74-2. Description of production apparatus)

As illustrated in FIG. 4, the production apparatus 200 that comprised the first container 110, the first supplying portion 120, the second container 130, the second supplying portion 140, the extraction duct 150 having the flow channel 170 (See FIG. 2) therein, the collector 160, the first circulation portion 280 and the second circulation portion 290, was prepared.

The jar fermentor was used as the first container 110.

The first supplying portion 120 was provided so as to be able to supply the first liquid 10 stored in the first container 110 to the T-shaped mixer 151 of the extraction duct 150 using a PFA tube (inner diameter 1.59 mm) as the first supplying duct 121 and a plunger pump as the first liquid feeding pump 122.

A container storing therein isopropyl myristate as the second liquid 20 was used as the second container 130.

The second supplying portion 140 was provided so as to be able to supply the second liquid 20 stored in the second container 130 to the T-shaped mixer 151 of the extraction duct 150 using the PFA tube (inner diameter 1.59 mm) as the second supplying duct 141 and the plunger pump as the second liquid feeding pump 142.

The extraction duct 150 was made of a member in which the PFA tube (inner diameter 1.59 mm) as a duct part 152 was connected to the T-shaped mixer 151. In the flow channel 170 inside the extraction duct 150, as illustrated in FIG. 2, the merging part 171 and the flow part 172 correspond to the part inside the T-shaped mixer 151 and the part inside the duct part 152, respectively.

The collector 160 was provided so as to be able to collect the first liquid 10 and the second liquid 20 that are discharged from the flow channel 170 at an outlet of the duct part 152.

The first circulation portion 280 was provided so as to be able to return the first liquid 10 stored in the collector 160 to the jar fermentor as the first container 110 with use of the duct as the first circulation duct 281 and the pump as the first circulation pump 282.

The second circulation portion 290 was provided so as to be able to return the second liquid 20 stored in the collector 160 to the second container 130 with use of the duct as the second circulation duct 291 and the pump as the second circulation pump 292.

### (74-3. Operation for producing linalool)

Microorganisms was cultured in the jar fermentor under the fermentation conditions described above.

The pump was actuated at 13.5 hours after the beginning of the culture when linalool began to be generated. For 11 hours until 24.5 hours after the beginning of the culture, the extraction operation was carried out as described below.

The culture medium (inside the jar fermentor, 30°C) was extracted at 2.5 mL/min as the first liquid from the jar fermentor and then supplied to the merging part inside the T-shaped mixer of the extraction duct. At the same time, isopropyl myristate (normal temperature) was extracted at 2.5 mL/min as the second liquid from the second container and then supplied to the merging part inside the T-shaped mixer of the extraction duct.

The culture medium and isopropyl myristate merged at the merging part, and passed through the flow part inside the PFA tube while forming slug flows. In the flow part, the culture medium and isopropyl myristate had liner velocity of 0.04 m/s and flowing time of 24 seconds. The temperature at the flow part was not particularly controlled.

Afterwards, the culture medium and isopropyl myristate were collected into the collector. In the collector, the phase separation was quickly caused such that the culture medium was present in a lower part while isopropyl myristate was present in an upper part. The temperature was not particularly controlled in the collector.

The culture medium was extracted from the lower part inside the collector and then returned to the jar fermentor for the circulation thereof. Isopropyl myristate was extracted from the upper part inside the collector and then returned to the second container for the circulation thereof.

In the above extraction operation, the flow amount of the culture medium returned to the jar fermentor from the collector was precisely controlled continuously while visually observed, for the purpose of shortening the staying time in the collector to avoid shortage of oxygen. The circulation time for allowing the culture medium to circulate outside of the jar fermentor was 4 minutes according to the estimation from the capacity of the duct and the flow amount.

In the aforementioned extraction operation, the amount of isopropyl myristate was 200 mL. Isopropyl myristate was replaced with 200 mL of new one at 19 hours after the beginning of the culture. Total amount of isopropyl myristate used was 400 mL.

### (74-4. Evaluation)

### 1. Evaluation of separability:

The culture medium and isopropyl myristate collected in the collector were visually observed to investigate whether the phase of the culture medium and the phase of isopropyl myristate were quickly separated.

### 2. Measurement of linalool extraction amount:

The culture medium and isopropyl myristate were sampled with time. The linalool concentrations in the sampled culture medium and isopropyl myristate were measured by gas chromatography. From the obtained concentration, the amount of linalool contained in each of the culture medium and the isopropyl myristate was calculated.

### 3. Measurement of organic acid concentration:

The organic acid concentration in the culture medium was measured at the end of extraction operation (24.5 hours after the beginning of the culture) in order to evaluate the influence of shortage of oxygen on the microorganism during the period of circulation outside the jar fermentor. The organic acid concentration was measured by high-speed liquid chromatography (HPLC) using a Prominence organic acid analysis system (Shimadzu corporation). When the microorganism is in oxygen-deficient condition, metabolic pathway of the microorganism is influenced to increase accumulated amounts of acetic acid and lactic acid.

### [Comparative Example 13]

The same operations were carried out in the same manner as in Example 74 to produce linalool by the culture, except that the extract operation was omitted. During the culture, the culture medium was sampled with time and the amount of linalool contained in the culture medium was obtained in the same method as in Example 74. In the same method as in Example 74, the concentration of organic acid in the culture medium was measured at 24.5 hours after the beginning of the culture.

### [Results of Example 74 and Comparative Example 13]

### 1. Result of separability:

In Example 74, the phase of the culture medium containing the microorganism and the phase of the isopropyl myristate containing no microorganism are separated from each other within several seconds after the collection into the collector. Therefore, Example 74 confirmed that the microorganism and the linalool can be separated in a short time.

### 2. Results of linalool extraction amount:

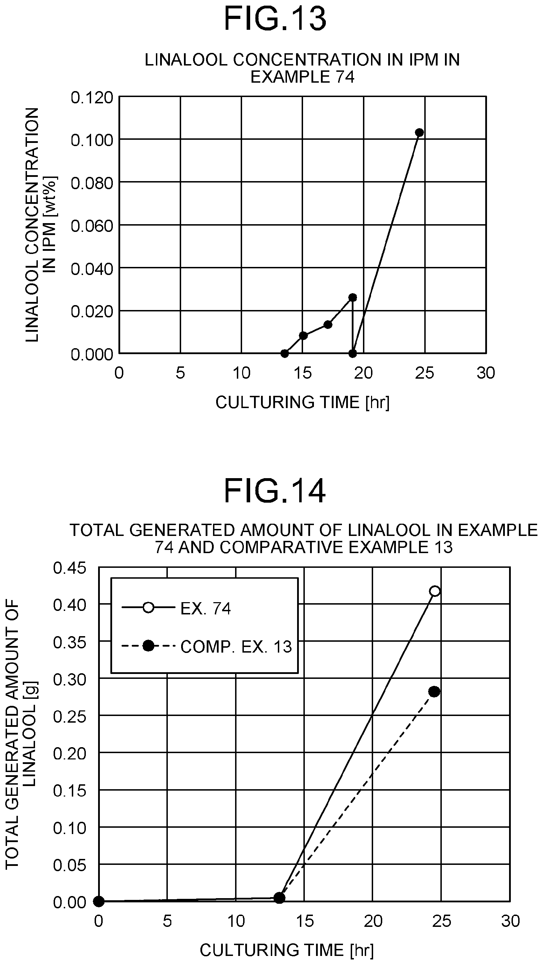

The linalool concentration in isopropyl myristate measured for Example 74 is illustrated in FIG. 13. As illustrated in FIG. 13, the linalool concentration of isopropyl myristate was increased after the beginning of the extraction operation. Thus, Example 74 confirmed that the culture can be continuously performed while extraction from the culture medium to isopropyl myristate is carried out.

FIG. 14 illustrates the total generated amount of linalool contained in the culture medium and the linalool contained in isopropyl myristate in Examples 74 and Comparative Example 13. As illustrated in FIG. 14, compared to Comparative Example 13, linalool is generated in higher amount in Example 74. The total generated amount of linalool at 24.5 hours after the beginning of culturing is 0.4 g in Example 74 and 0.28 g in Comparative Example 13. Results of analysis reveals that the linalool concentration of the culture medium in the jar fermentor can be suppressed in Example 74, compared to Comparative Example 13. Thus, Example 74 enables the linalool production using microorganism while avoiding growth inhibition caused by linalool.

### 3. Results of organic acid concentration:

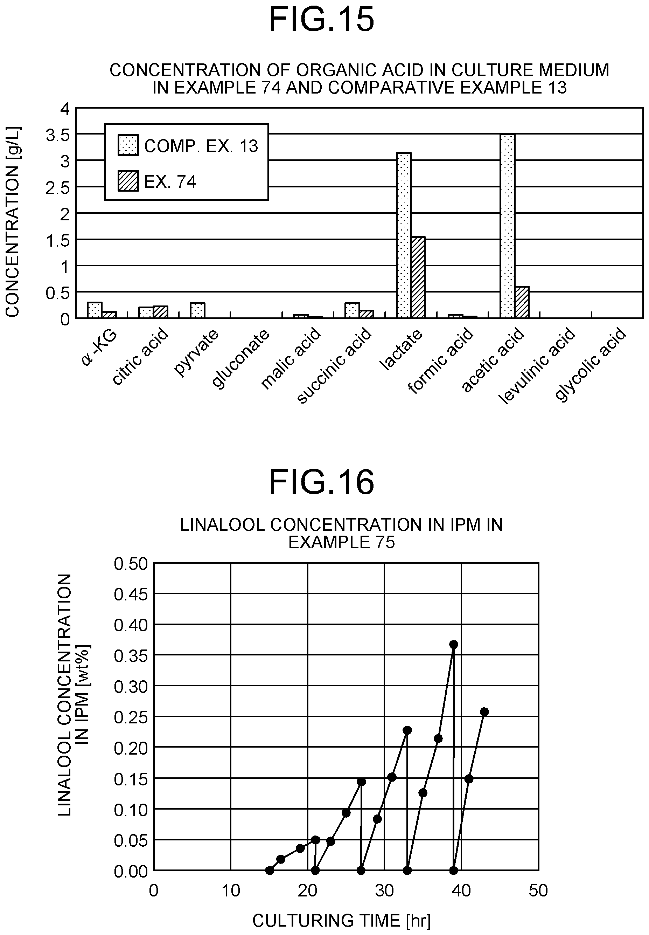

FIG. 15 illustrates the organic acid concentrations of the culture mediums at 24.5 hours after the beginning of culturing in Example 74 and Comparative Example 13. As illustrated in FIG. 15, remarkable accumulation of acetic acid and lactic acid was not observed in the culture medium in Example 74. Therefore, Example 74 suggest that shortage of oxygen is not caused.

### [Example 75]

### (75-1. Fermentation condition)

Jar culture was carried out using Pantoea ananatis SWITCH-PphoCΔgcd/AaLINS-ispA* strain (WO2017/051928). For the jar culture, the jar fermentor (ABLE Corporation) was used as the fermentation tank with a 1 L capacity. Glucose medium was prepared according to the composition listed in Table 9.

The SWITCH-PphoCΔgcd/AaLINS-ispA* strain, an isoprene-generating microorganism, was applied on the LB plate containing kanamycin (50 mg/L) and then cultured at 34°C for 16 hours as the pre-culture. Next, 300 mL of the glucose medium (Table 9) described below was put into the jar fermentor with a 1 L capacity, and then one plate of sufficiently grown bacterial cells were inoculated for starting the culture. The culture temperature was set at 34°C for the first 11 hours, and 30°C afterward. pH for the culture was controlled to 6.8 with use of ammonia gas. Ventilation was carried out at 300 mL/minute. Dissolved oxygen concentration was set to 21% at the beginning of the culture prior to the inoculation. Dissolved oxygen concentration was set to 0% in saturated sodium sulfite solution. Stirring was controlled to maintain a dissolved oxygen concentration of 5% or more in the culture medium with use of the galvanic type sensor (ABLE Corporation). A feed medium listed in Table 10 was added at 1.9 mL per hour to maintain the glucose concentration in a range of 10 to 50 g/L in the medium.

### [Table 9]

**Table 9. Composition of Glucose Media**

| Composition A | (concentration) |
|---|---|
| Glucose | 120 g/L |
| MgSO₄.7aq | 2.2 g/L |

| Composition B | |
|---|---|
| Trisodium citrate | 2.0g/L |
| (NH₄)₂SO₄ | 2.0g/L |
| KH₂PO₄ | 4.0g/L |
| Yeast Extract | 4.0g/L |
| FeSO₄.7aq | 20mg/L |
| MnSO₄.5aq | 20mg/L |
| DISFOAM GD-113K | 0.02 mL/L |

After 0.135 L of compositions were prepared for a composition A and a composition B (without pH adjustment), the compositions were sterilized by heat at 120°C for 20 minutes. After cooled down, the compositions for compositions A and B were mixed at 1:1. Then, kanamycin (100 mg/L in final concentration) was added to the resulting mixture. In Example 75, 30 mL of ultra-pure water was added right before the beginning of culturing, for use as a medium.

### [Table 10]

**Table 10. Composition of Feed Media**

| Feed Media | (concentration) |
|---|---|
| Glucose | 700 g/L |
| DISFOAM GD-113K | 0.7 mL/L |

After prepared, the 1 L of medium (without pH adjustment) was sterilized by heat at 120°c for 20 minutes.

After the beginning of culturing, samples were taken as appropriate for measurement of O.D. value and GC analysis of linalool. The standard solution of linalool was prepared from the reagent linalool (manufactured by Wako Pure Chemical Corporation). The sample to be used for the measurement was diluted with ethanol (manufactured by Wako Pure Chemical Corporation) as appropriate.

GC analysis and the organic acid analysis were carried out in the same method as in Example 74.

### (75-2. Description of production apparatus)

The same production apparatus as that of Example 74 was used.

### (75-3. Operation for producing linalool)

Microorganisms was cultured in the jar fermentor under the fermentation conditions described above.

The pump was actuated at 15 hours after the beginning of culturing when linalool began to be generated. For 28 hours until 43 hours after the beginning of culturing, the extraction operation was carried out as described below.

The culture medium (inside the jar fermentor, 30°C) was extracted at 2.5 mL/min as the first liquid from the jar fermentor and then supplied to the merging part inside the T-shaped mixer of the extraction duct. At the same time, isopropyl myristate (normal temperature, no control) was extracted at 2.5 mL/min as the second liquid from the second container and then supplied to the merging part inside the T-shaped mixer of the extraction duct.

The culture medium and isopropyl myristate merged at the merging part, and passed through the flow part inside the PFA tube while forming slug flows. In the flow part, the culture medium and isopropyl myristate had linear velocity of 0.04 m/s and flowing time of 24 seconds. The temperature at the flow part was not particularly controlled.

Afterwards, the culture medium and isopropyl myristate were collected into the collector. In the collector, the phase separation was quickly caused such that the culture medium was present in the lower part while isopropyl myristate was present in the upper part. The temperature was not particularly controlled in the collector.

The culture medium was extracted from the lower part inside the collector and then returned to the jar fermentor for the circulation thereof. Isopropyl myristate was extracted from the upper part inside the collector and then returned to the second container for the circulation thereof.

In the aforementioned extraction operation, the flow amount of the culture medium returned to the jar fermentor from the collector was precisely controlled constantly while visually observed, for the purpose of shortening the staying time in the collector to avoid shortage of oxygen. The circulation time for allowing the culture medium to circulate outside the jar fermentor was 4 minutes according to the estimation from the capacity of the duct and the flow amount.

In the aforementioned extraction operation, the amount of isopropyl myristate was 100 mL. Isopropyl myristate was replaced with 100 mL of new one at 21, 27, 33 and 39 hours after the beginning of culturing. Total amount of isopropyl myristate used was 500 mL.

### (75-4. Evaluation)

The evaluation of separability, measurement of linalool extraction amount and measurement of organic acid concentration were carried out in the same method as in Example 74. The organic acid concentration was measured for the culture medium at the end of extraction operation (43 hours after the beginning of culturing).

### [Comparative Example 14]

To the medium obtained by mixing the composition A with the composition B, 30 mL (10 vol%) of isopropyl myristate was added instead of 30 ml of ultra-pure water right before the beginning of culturing. The extraction operation was not performed. Except for the aforementioned procedure, linalool was produced by the culture in the same operation as in Example 75. During the culture, the culture medium was sampled with time to determine the amount of linalool contained in the culture medium in the same method as in example 75. The organic acid concentration of the culture medium was measured at 43 hours after the beginning of culturing in the same method as in Example 75.

### [Comparative Example 15]

To the medium obtained by mixing the composition A with the composition B, 6 mL (2 vol%) of isopropyl myristate and 24 mL of ultra-pure water were added instead of 30 mL of ultra-pure water right before the beginning of culturing. The extraction operation was not performed. Except for the aforementioned procedure, linalool was produced by the culture in the same operation as in Example 75. During the culture, the culture medium was sampled with time to determine the amount of linalool contained in the culture medium in the same method as in Example 75. The organic acid concentration of the culture medium was measured at 43 hours after the beginning of culturing in the same method as in Example 75.

### [Comparative Example 16]

To the medium obtained by mixing the composition A with the composition B, 1.5 mL (0.5 vol%) of isopropyl myristate and 28.5 mL of ultra-pure water were added instead of 30 mL of ultra-pure water right before the beginning of culturing. The extraction operation was not performed. Except for the aforementioned procedure, linalool was produced by the culture in the same operation as in Example 75. However, the culture was terminated at 39 hours after the beginning of culturing because of vigorous bubbling during the culture. During the culture, the culture medium was sampled with time to determine the amount of linalool contained in the culture medium in the same method as in Example 75. The organic acid concentration of the culture medium was measured at 39 hours after the beginning of culturing in the same method as in Example 75.

### [Results of Example 75 and Comparative Examples 14-16]

### 1. Results of separability:

In Example 75, the phase of the culture medium containing the microorganism and the phase of the isopropyl myristate containing no microorganism are separated from each other within several seconds after the collection into the collector. Therefore, Example 75 confirmed that the microorganism and the linalool can be separated from each other within a short time.

### 2. Results of linalool extraction amount:

FIG. 16 illustrates the linalool concentration in isopropyl myristate measured for Example 75. As illustrated in FIG. 16, the linalool concentration of isopropyl myristate is increased after the beginning of the extraction operation. Thus, Example 75 confirmed that the culture can be continuously performed while extraction from the culture medium to isopropyl myristate is carried out.

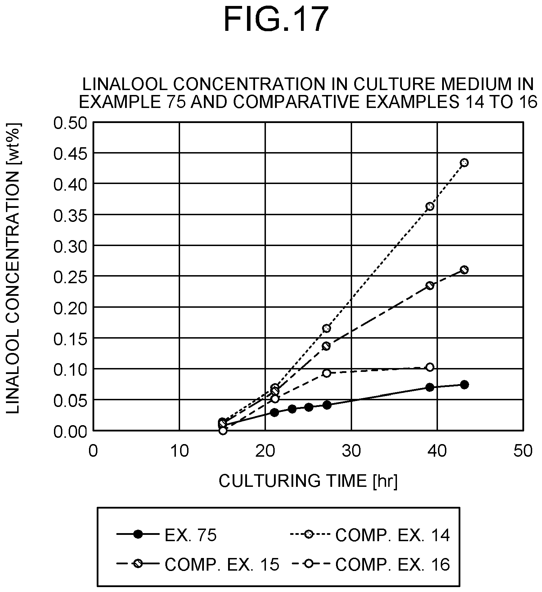

FIG. 17 illustrates the linalool concentration in the culture medium in Example 75 and Comparative Examples 14 to 16. As illustrated in FIG. 17, in Example 75, the linalool concentration of the culture medium in the jar fermentor can be suppressed. In view of the increase in the linalool concentration of isopropyl myristate as illustrated in FIG. 16, Example 75 was proved to enable the linalool production using microorganism while avoiding growth inhibition.

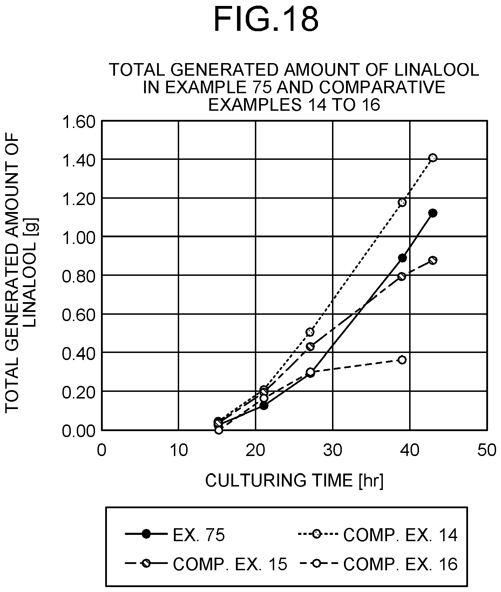

FIG. 18 illustrates the total generated amount of the linalool contained in the culture medium and the linalool contained in isopropyl myristate in Example 75 and Comparative Examples 14 to 16. As illustrated in FIG. 18, in Example 75, a larger amount of linalool can be produced until the end of culturing. The total production amount of linalool at the end of culturing is 1.1 g in Example 75, 1.4 g in Comparative Example 14, 0.9 g in Comparative Example 15, and 0.4 g in Comparative Example 16.

### 3. Results of organic acid concentration:

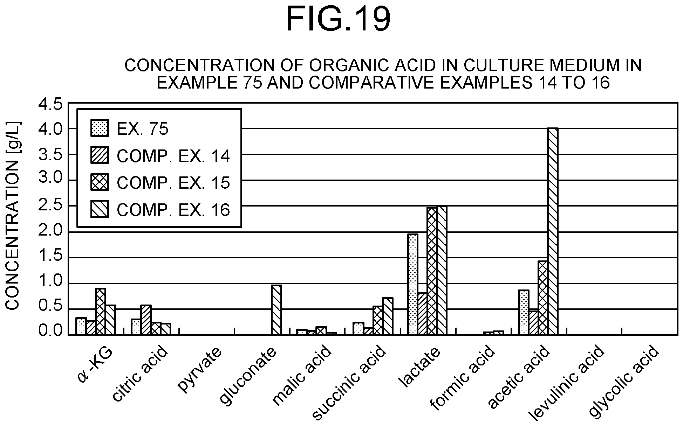

FIG. 19 illustrates the organic acid concentration in the culture medium at 43 hours after the beginning of culturing in Example 75 and Comparative Examples 14 to 15 as well as the organic acid concentration in the culture medium at 39 hours after the beginning of culturing in Comparative Example 16. As illustrated in FIG. 19, in Example 75, remarkable accumulation of lactic acid and acetic acid is not observed in the culture medium. Therefore, Example 75 was suggested to be free from oxygen deficiency.

### 4. Investigation:

In Example 75, the concentration of isopropyl myristate in the culture medium at 43 hours after the beginning of culturing was confirmed to be 0.2 weight%. This concentration is lower than the isopropyl myristate concentrations in the culture mediums in the batch two-phase fermentation of Comparative Examples 14 to 16. Such a low concentration of isopropyl myristate is not expected to alleviate the growth inhibition inside the jar fermentor. Therefore, the continuous linalool production in Example 75 is expected to come from an effect resulting from the extraction outside the jar fermentor.

### [Reference Example 1]

In Example 75, after the extraction operation was terminated, the culture was further continued inside the jar fermentor. As a result, the O.D. 600 nm of the culture medium was decreased down to 33.6 at the end of culturing for 68.5 hours.

### Explanation of references

- 10: first liquid
- 20: second liquid
- 30: phase of first liquid
- 40: phase of second liquid
- 100: extraction apparatus
- 110: first container
- 120: first supplying portion
- 121: first supplying duct
- 122: first liquid feeding pump
- 130: second container
- 140: second supplying portion
- 141: second supplying duct
- 142: second liquid feeding pump
- 150: extraction duct
- 151: T-shaped mixer part
- 152: duct part
- 160: collector
- 170: flow channel
- 171: merging part
- 172: flow part
- 173: outlet part
- 200: production apparatus
- 280: first circulation portion
- 281: first circulation duct
- 282: first circulation pump
- 290: second circulation duct
- 291: second circulation duct
- 292: second circulation pump

## Claims

1. An extraction method for extracting an object component by transferring the object component from a first liquid containing a microorganism and the object component to a second liquid to which the object component is transferrable and that is capable of causing phase separation from the first liquid, with use of a flow channel including a merging part, a flow part, and an outlet part, the extraction method comprising:
a step of supplying the first liquid to the merging part;
a step of supplying the second liquid to the merging part;
a step of passing the first liquid and the second liquid supplied to the merging part through the flow part, in a state where a phase of the first liquid and a phase of the second liquid are separated from each other;
a step of discharging the first liquid and the second liquid having passed through the flow part, from the outlet part; and
a step of collecting the first liquid and the second liquid having been discharged from the outlet part, into a collector.

2. The extraction method according to claim 1, wherein, in the flow part, the first liquid and the second liquid form slug flow with an alternate alignment of the phase of the first liquid and the phase of the second liquid in a flowing direction.

3. The extraction method according to claim 1 or 2, wherein the microorganism has an ability to generate the object component.

4. The extraction method according to any one of claims 1 to 3, wherein the microorganism is a genetically modified microorganism.

5. The extraction method according to any one of claims 1 to 4, wherein the first liquid is a culture medium containing the microorganism and the object component, and the second liquid contains an organic solvent.

6. The extraction method according to any one of claims 1 to 5, wherein the object component is a hydrophobic substance.

7. The extraction method according to any one of claims 1 to 6, wherein the object component is an isoprenoid compound or a vanilloid compound.

8. The extraction method according to any one of claims 1 to 7, wherein the microorganism is a bacterium or a yeast.

9. The extraction method according to claim 8, wherein the bacterium is selected from the group consisting of Corynebacterium bacterium, Pantoea bacterium, Bacillus bacterium, Escherichia bacterium, and Enterobacter bacterium.

10. The extraction method according to any one of claims 1 to 9, wherein a concentration of the object component in the first liquid supplied to the merging part is 0.1 g/L or more and 500 g/L or less.

11. The extraction method according to any one of claims 1 to 10, wherein a dry microorganism weight of the first liquid supplied to the merging part is 0.1 g/L or more and 500 g/L or less.

12. The extraction method according to any one of claims 1 to 11, wherein the flow part has an inner diameter of 0.25 mm or more and 10.0 mm or less.

13. The extraction method according to any one of claims 1 to 12, wherein temperatures of the first liquid and the second liquid in the flow part are 10°C or more and 90°C or less.

14. An extraction apparatus for extracting an object component by transferring the object component from a first liquid containing a microorganism and the object component to a second liquid to which the object component is transferrable and that is capable of causing phase separation from the first liquid, the extraction apparatus comprising:
a first supplying portion that can supply the first liquid;
a second supplying portion that can supply the second liquid;
a flow channel including
a merging part to which the first liquid and the second liquid can be supplied,
a flow part through which the first liquid and the second liquid supplied to the merging part can pass, and
an outlet part from which the first liquid and the second liquid passed through the flow part can be discharged; and
a collector that can collect the first liquid and the second liquid discharged from the outlet part, wherein
the first liquid and the second liquid can pass through the flow part in a state where a phase of the first liquid and a phase of second liquid are separated from each other.

15. A production method for producing an object component, the production method comprising:
a step of generating the object component by a microorganism in a first container that stores therein a first liquid containing the microorganism;
a step of supplying the first liquid from the first container to a merging part of a flow channel that includes the merging part, a flow part, and an outlet part;
a step of supplying a second liquid to which the object component is transferrable and that is capable of causing phase separation from the first liquid, to the merging part;
a step of passing the first liquid and the second liquid supplied to the merging part through the flow part, in a state where a phase of the first liquid and a phase of the second liquid are separated from each other;
a step of discharging the first liquid and the second liquid having passed through the flow part, from the outlet part; and
a step of collecting the first liquid and the second liquid having been discharged from the outlet part, into a collector.

16. The production method for the object component according to claim 15, including a step of returning the first liquid collected in the collector to the first container.

17. The production method for the object component according to claim 15 or 16, including a step of returning the second liquid collected in the collector to the merging part.

18. The production method for the object component according to any one of claims 15 to 17, wherein the object component has a property of inhibiting the microorganism from generating the object component.

19. A production apparatus for producing an object component, the production apparatus comprising:
a first container that can store a first liquid containing a microorganism and can allow the microorganism to generate the object component;
a first supplying portion that can supply the first liquid stored in the first container;
a second supplying portion that can supply a second liquid to which the object component is transferrable and that is capable of causing phase separation from the first liquid;
a flow channel including
a merging part to which the first liquid and the second liquid can be supplied,
a flow part through which the first liquid and the second liquid supplied to the merging part can pass, and
an outlet part from which the first liquid and the second liquid passed through the flow part can be discharged; and
a collector that can collect the first liquid and the second liquid discharged from the outlet part, wherein
the first liquid and the second liquid can pass through the flow part in a state where a phase of the first liquid and a phase of second liquid are separated from each other.

20. The production apparatus for the object component according to claim 19, comprising a first circulation portion that can return the first liquid collected in the collector to the first container.
